(19) Europäisches Patentamt
European Patent Office
Office européen des brevets

(11) **EP 4 595 870 A1**

(12) **EUROPEAN PATENT APPLICATION**
published in accordance with Art. 153(4) EPC

(43) Date of publication:
06.08.2025 Bulletin 2025/32

(21) Application number: 23872057.7

(22) Date of filing: 20.09.2023

(51) International Patent Classification (IPC):
*A61B 3/08* (2006.01)

(52) Cooperative Patent Classification (CPC):
A61B 3/08

(86) International application number:
PCT/JP2023/034003

(87) International publication number:
WO 2024/070829 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.09.2022 JP 2022155540

(71) Applicant: Topcon Corporation
Tokyo 174-8580 (JP)

(72) Inventor: INUZUKA, Naoki
Tokyo 174-8580 (JP)

(74) Representative: Louis Pöhlau Lohrentz
Patentanwälte
Merianstrasse 26
90409 Nürnberg (DE)

(54) **OPHTHALMOLOGICAL DEVICE**

(57) To provide an ophthalmologic apparatus capable of objectively determining positional relationship between a subject eye and an acquisition optical system even when the subject places his/her face against a head portion. The ophthalmologic apparatus (10) includes a pair of acquisition optical systems (measurement optical systems 20) that are configured to acquire eye information of both subject eyes E, an optical system change mechanism (measurement head driver 15) that is configured to change at least one of a position or an orientation of a pair of acquisition optical systems (measurement optical systems 20), and a controller (27) that is configured to create a positional relationship image Ip showing a positional relationship of a pair of acquisition optical systems with respect to a pair of subject eyes based on optical posture information indicating the position and the orientation of a pair of acquisition optical systems (measurement optical system 20) set by the optical system change mechanism (measurement head driver 15) and subject eye position information indicating a position of the subject eye, and displays the positional relationship image on a display (34).

[FIG.4]

EP 4 595 870 A1

# Description

TECHNICAL FIELD

This disclosure relates to an ophthalmologic apparatus.

BACKGROUND

Ophthalmologic apparatuses are conventionally known that can examine (optometry) the visual function of subject eyes in binocular vision by simultaneously acquiring information on both subject eyes with a measurement optical system and based on a response from a subject regarding a visibility of a visual target presented to both subject eyes (see, for example, Patent Literature 1).

This ophthalmologic apparatus includes a head portion provided with the measurement optical system. A subject places his/her face against the head portion to be placed in an appropriate posture. The ophthalmologic apparatus thus can properly acquire the information on the subject eyes and can properly examine the subject eyes based on the visibility of the presented visual target.

CITATION LIST

Patent Literature

Patent Literature: International Publication No. 2003/041571

SUMMARY

Technical Problem

The ophthalmologic apparatus includes the head portion to which the subject appropriately puts his/her face and with which the measurement optical system is provided, which makes it difficult to visually observe the subject eyes and the measurement optical system. Therefore, the ophthalmologic apparatus cannot objectively determine the positional relationship between the subject eyes and an acquisition optical system, which makes it difficult to confirm the condition of the test (optometry) of the visual function of the subject eyes.

The present disclosure has been made in view of the above circumstances, and the object of the present disclosure is to provide an ophthalmologic apparatus that can objectively determine the positional relationship between a subject eye and an acquisition optical system even when a subject places his/her face against a head portion.

Solution to Problem

To solve the above problem, an ophthalmologic apparatus of the present disclosure includes a pair of acquisition optical systems that are configured to acquire eye information of both subject eyes, an optical system change mechanism that is configured to change at least one of a position or an orientation of a pair of acquisition optical systems, and a controller that is configured to create a positional relationship image showing a positional relationship of the pair of acquisition optical systems with respect to a pair of subject eyes based on optical posture information indicating the position and the orientation of a pair of acquisition optical systems set by the optical system change mechanism and subject eye position information indicating a position of the subject eye, and displays the positional relationship image on a display.

Advantageous Effect

The ophthalmologic apparatus of the present disclosure allows the examiner to objectively understand the positional relationship between the subject eye and the acquisition optical system even when the subject places his/or her face against the head portion.

BRIEF DESCRIPTION OF THE DRAWING

[FIG. 1] FIG. 1 is an explanatory view illustrating an overall configuration of an ophthalmologic apparatus of Embodiment 1 as an example of an ophthalmologic apparatus according to the present disclosure.
[FIG. 2] FIG. 2 is an explanatory view schematically illustrating a configuration in which a pair of measurement heads is movable via a drive mechanism in the ophthalmologic apparatus.
[FIG. 3] FIG. 3 is an explanatory view illustrating a schematic configuration of a measurement optical system of the ophthalmologic apparatus.
[FIG. 4] FIG. 4 is a block diagram illustrating a configuration of a control system of the ophthalmologic apparatus.
[FIG. 5] FIG. 5 is an explanatory view illustrating the configuration of the measurement optical system.
[FIG. 6] FIG. 6 is a schematic view illustrating a configuration example of a conical prism used in the ophthalmologic apparatus of Embodiment 1.
[FIG. 7] FIG. 7 is a schematic view illustrating a configuration example of a field lens used in the ophthalmologic apparatus.
[FIG. 8] FIG. 8 is an explanatory view illustrating a relationship between a focusing distance, a rotation angle, and a size of a visual target image on a display in relation to changes in a presentation position.
[FIG. 9] FIG. 9 is an explanatory view illustrating the relationship between the presentation position, the focusing distance, and the rotation angle.
[FIG. 10] FIG. 10 is a view illustrating an example of an adjustment force measurement screen displayed on a display of the ophthalmologic apparatus.

[FIG. 11] FIG. 11 is a view explaining an angle between an optical axis and a pupil axis.

[FIG. 12] FIG. 12 is an explanatory view illustrating a positional relationship image.

[FIG. 13] FIG. 13 is an explanatory view illustrating a positional relationship image in an example different from FIG. 12.

[FIG. 14] FIG. 14 is an explanatory view illustrating a characteristic configuration of an ophthalmologic apparatus in a modified example.

[FIG. 15] FIG. 15 is an explanatory view illustrating an example of arrangement of a measurement head in the ophthalmologic apparatus of the modified example.

[FIG. 16] FIG. 16 is an explanatory view illustrating a far-point position and a near-point position of a bifocal lens.

[FIG. 17] FIG. 17 is an explanatory view illustrating the positional relationship image in the ophthalmologic apparatus of the modified example.

[FIG. 18] FIG. 18 is an explanatory view illustrating an overall configuration of an ophthalmologic apparatus in Embodiment 2.

[FIG. 19] FIG. 19 is an explanatory view illustrating a positional relationship image in an example different from FIGS. 12, 13.

DESCRIPTION OF EMBODIMENTS

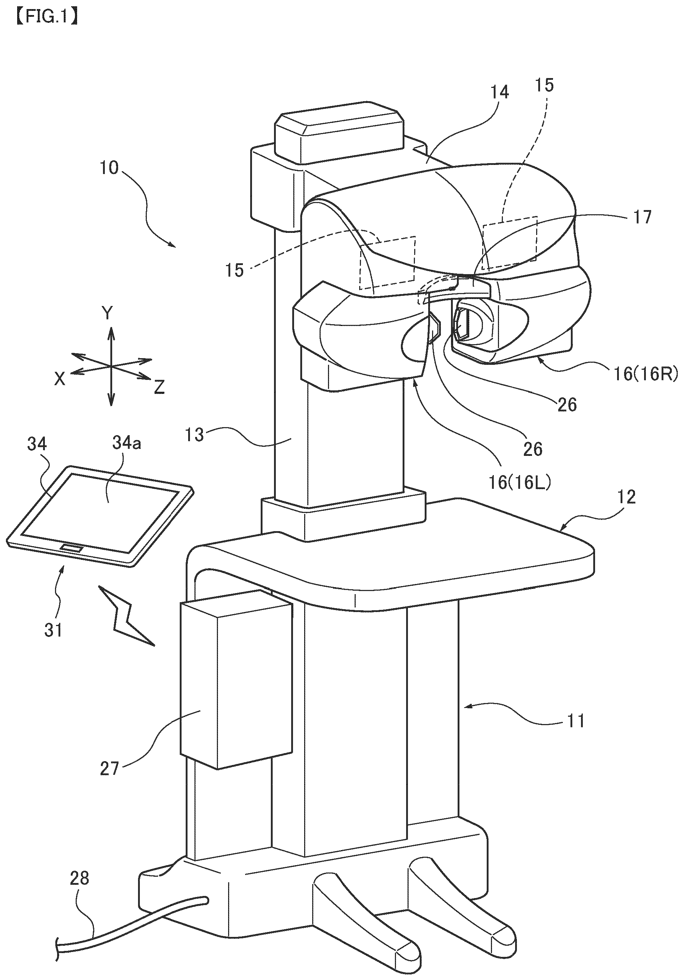

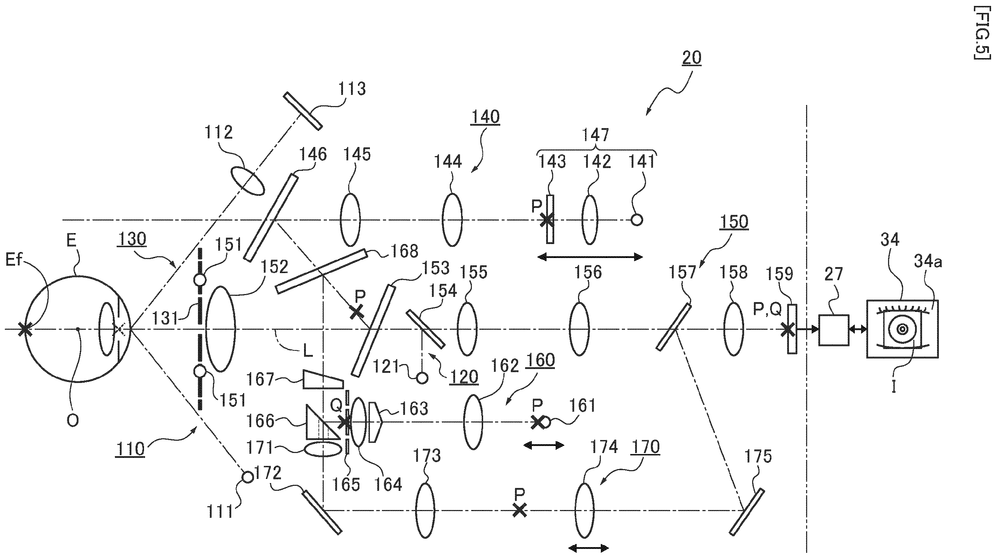

Embodiments of ophthalmologic apparatuses of the present disclosure will be described below with reference to FIGS. 1-19. FIG. 5 omits a deflection member 26 to easily understand the configuration and the contents illustrated in FIG. 5.

[Embodiment 1]

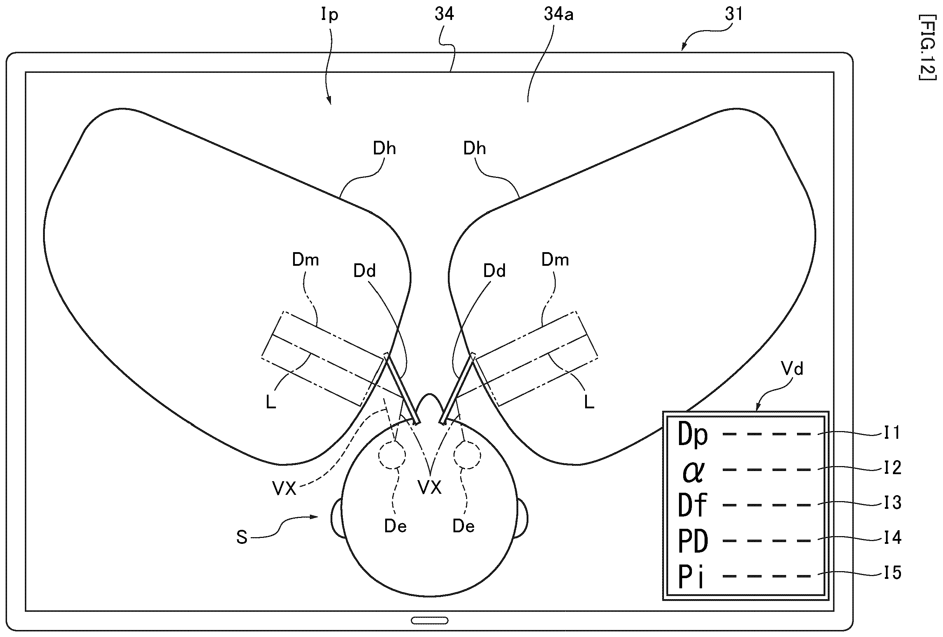

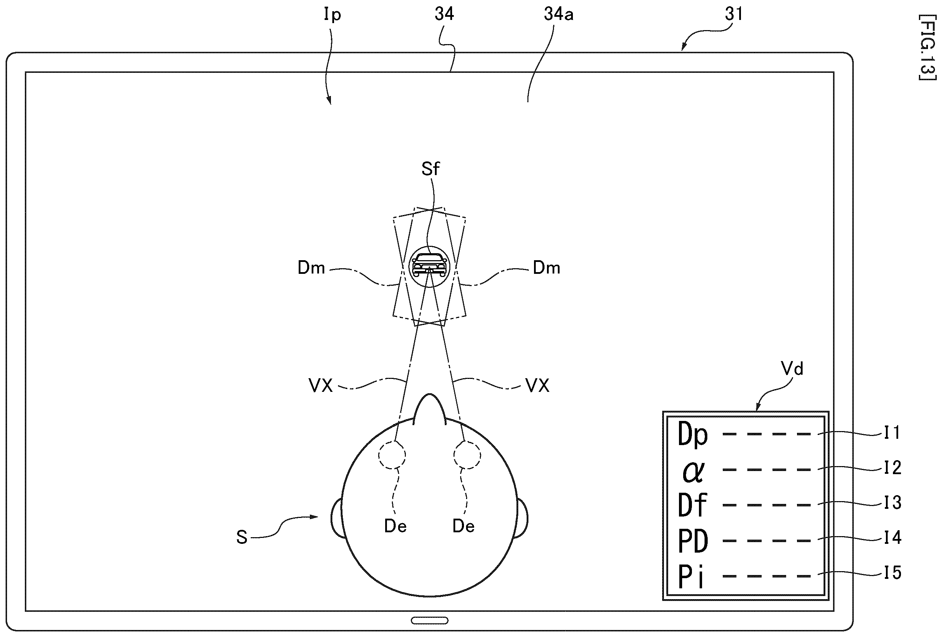

An ophthalmologic apparatus 10 of the present disclosure acquires eye information of a subject eye E of a subject S (see FIGS. 12, 13). The ophthalmologic apparatus 10 of Embodiment 1 is a binocular open type ophthalmologic apparatus that can simultaneously measure the characteristics of both subject eyes E with both eyes of the subject S open. The ophthalmologic apparatus 10 of Embodiment 1 may also examine the subject eyes independently by shielding one eye or turning off a fixed visual target.

The ophthalmologic apparatus 1 can perform both of any subjective examination and any objective examination. The subjective examination includes a subjective refraction measurement such as a far-point test, an intermediate-point test, a near-point test, a contrast test, and a glare test, an RG test, and a visual field test. In the objective examination, the ophthalmologic apparatus 10 irradiates light to the subject eye E and measures (acquires) information (eye characteristics) on the subject eye E based on the detection result of the returned light. The objective examination includes a measurement that acquires the characteristics of the subject eye E and imaging that captures the image of the subject eye E. Additionally, the objective examination includes an objective refraction measurement (refraction measurement), a corneal shape measurement (keratometry measurement), an intraocular pressure measurement, ocular fundus imaging, tomogram imaging using an optical coherence tomography (hereinafter referred to as OCT) (OCT imaging), and a measurement using an OCT.

As shown in FIG 1, the ophthalmologic apparatus 10 includes a support base 11, an optometry table 12, a column 13, a support arm 14, a pair of measurement head drivers 15, and a pair of measurement heads 16. The ophthalmologic apparatus 10 acquires information of the subject eye E of the subject S when the subject S facing the optometry table 12 places his/her forehead on a forehead rest 17 provided between both measurement heads 16. In the following, from the perspective of the subject S facing the ophthalmologic apparatus 10, the left and right direction is indicated with an arrow X, the up and down direction (vertical direction) is indicated with an arrow Y, and the direction orthogonal to the left and right direction and the up and down direction (depth direction of ophthalmologic apparatus 10) is indicated with an arrow Z as the front and back direction.

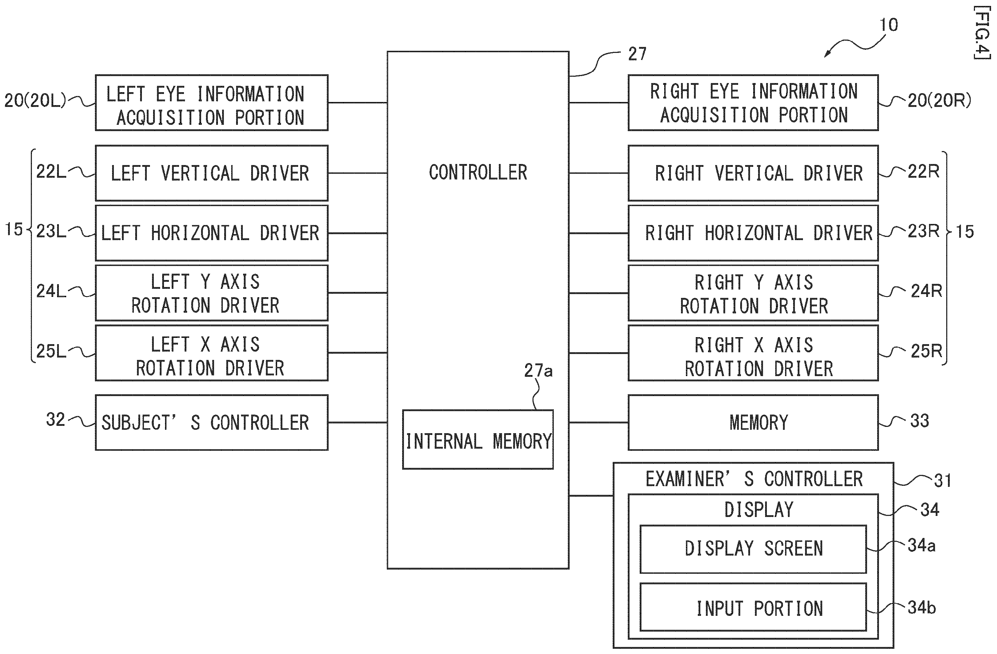

The optometry table 12 is supported by the support base 11. The optometry table 12 may be supported by the support base 11 to adjust the position in the up and down direction (height position). This optometry table 12 may be used to place an after-described examiner's controller 31, an after-described subject's controller 32 (see FIG. 4), and a device and a tool for use in optometry.

The column 13 is supported by the support base 11 to extend in the up and down direction at the rear end of the optometry table 12 in the front and back direction, and includes at the upper end a beam-like support arm 14. The support arm 14 suspends both measurements heads 16 via the measurement head driver 15, and extends from the column 13 to the front side in the front and back direction. The support arm 14 may be movable in the up and down direction to the column 13 or may be movable in the left and right direction and the front and back directions to the column 13. The support arm 14 extends in the front and back direction from the top of the column 13 to the front side, and suspends a pair of measurement head drivers 15 at the front end to place a pair of measurement head drivers 15 on the optometry table 12. Each of the measurement head drivers 15 suspends the measurement head 16 to be movable. Thus, the support arm 14 suspends and supports a pair of measurement head drivers 15 and a pair of measurement heads 16.

The measurement head drivers 15 and the measurement heads 16 are paired in the left and right direction for each of the left and right subject eyes E of the subject S. The measurement head drivers 15 are referred to as a left eye measurement head driver 15L and a right eye measurement head driver 15R, and the measurement heads 16 are referred to as a left eye measurement head 16L and a right eye measurement head 16R when the measurement head drivers 15 and the measurement heads 16 are described individually. The left eye measurement head 16L acquires the information of the left subject eye E of the subject S, and the right eye measurement head 16R acquires the information of the right subject eye E of the subject S. The left eye measurement head driver 15L and the right eye measurement head driver 15R, and the left eye measurement head 16L and the right eye measurement head 16R are configured to be plane symmetrical relative to the vertical plane located in the middle between them in the left and right direction.

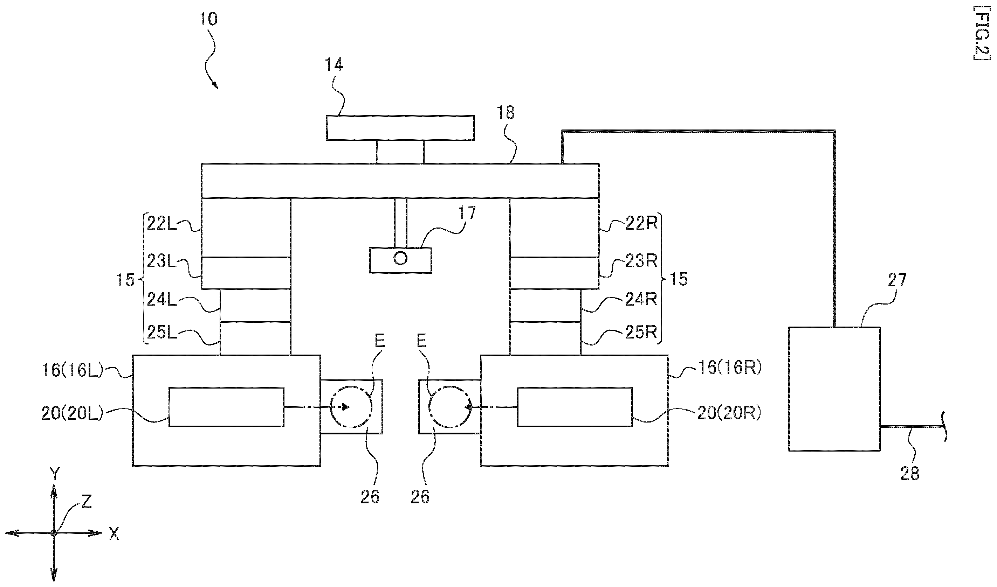

As shown in FIG 2, both measurement heads 16 are suspended to be movable by the measurement head drivers 15 via an attachment base 18 provided at the end of the support arm 14. In Embodiment 1, the measurement head driver 15 includes a left vertical driver 22L, a left horizontal driver 23L, a left Y-axis rotation driver 24L, and a left X-axis rotation driver 25L for the left eye measurement head 16L, and a right vertical driver 22R, a right horizontal driver 23R, a right Y-axis rotation driver 24R, and a right X-axis rotation driver 25R for the right eye measurement head 16R. The configuration of each driver for the left eye measurement head 16L and the configuration of each driver for the right eye measurement head 16R are plane symmetrical relative to the vertical plane located in the middle between them in the X direction, and are simply described as a vertical driver 22, a horizontal driver 23, a Y-axis rotation driver 24, and an X-axis rotation driver 25 except when these are described individually. The measurement head driver 15 has the following order, from the support arm 14 side, the vertical driver 22, the horizontal driver 23, the Y-axis rotation driver 24, and the X-axis rotation driver 25.

The attachment base 18 is fixed to the end of the support arm 14 and extends in the X direction. One end of the attachment base 18 suspends the left vertical driver 22L, the left horizontal driver 23L, the left Y-axis rotation driver 24L, and the left X-axis rotation driver 25L, and the other end of the attachment base 18 suspends the right vertical driver 22R, the right horizontal driver 23R, the right Y-axis rotation driver 24R, and the right X-axis rotation driver 25R. The center of the attachment base 18 suspends the forehead rest 17.

The vertical driver 22 is provided between the attachment base 18 and the horizontal driver 23, and moves the horizontal driver 23 relative to the attachment base 18 in the Y direction (vertical direction). The horizontal driver 23 is provided between the vertical driver 22 and the Y-axis rotation driver 24, and moves the Y-axis rotation driver 24 relative to the vertical driver 22 in the X and Z directions (horizontal direction). The vertical driver 22 and the horizontal driver 23 include an actuator that generates a driving force such as a pulse motor and a transmission mechanism that transmits a driving force such as a combination of gears or a rack- and-pinion. For example, providing the combination of the actuator and the transmission mechanism individually in the X direction and the Z direction can make it easy to configure the horizontal driver 23 and control the movement of the horizontal driver 23 in the horizontal direction.

The Y-axis rotation driver 24 is provided between the horizontal driver 23 and the X-axis rotation driver 25, and rotates the X-axis rotation driver 25 relative to the horizontal driver 23 about an eyeball rotation axis in the Y-axis direction that extends in the Y direction through the eyeball rotation point of the corresponding subject eye E. The X-axis rotation driver 25 is provided between the Y-axis rotation driver 24 and the corresponding measurement head 16, and rotates the corresponding measurement head 16 relative to the Y-axis rotation driver 24 about an eyeball rotation axis in the X-axis direction that extends in the X direction through the eyeball rotation point of the corresponding subject eye E. The Y-axis rotation driver 24 and the X-axis rotation driver 25 include an actuator and a transmission mechanism similar to the vertical driver 22 and the horizontal driver 23, and the transmission mechanism that receives the driving force from the actuator moves along a circular arc guide groove. The center position of the guide groove is aligned with the eyeball ration axis in the Y-axis direction. The Y-axis rotation driver 24 can thus rotate the measurement head 16 about the eyeball rotation axis of the subject eye E in the Y-axis direction. The center position of the guide groove is aligned with the eyeball rotation axis in the X-axis direction. The X-axis rotation driver25 can thus rotate the measurement head 16 about the eyeball rotation axis of the subject eye E in the X-axis direction. In other words, the alignment of each of the center positions of the guide grooves of the Y-axis rotation driver 24 and the X-axis rotation driver 25 with the eyeball rotation point of the subject eye E can rotate the measurement head 16 about the eyeball rotation point of the subject eye E in the left and right direction (rotation direction about Y direction) and the up and down direction (rotation direction about the X direction).

The Y-axis rotation driver 24 rotates the measurement head 16 while changing the position that supports the measurement head 16 to be rotatable about the Y-axis rotation axis and supporting the measurement head 16 via the X-axis rotation driver 25 in cooperation with the horizontal driver 23 to rotate the measurement head 16 about the eyeball rotation axis of the subject eye E in the Y-axis direction. The X-axis rotation driver 25 rotates the measurement head 16 while changing the position that supports the measurement head 16 to be rotatable about the X-axis rotation axis and supporting the measurement head 16 in cooperation with the vertical driver 22 to rotate the measurement head 16 about the eyeball rotation axis of the subject eye E in the X-axis direction.

This allows the measurement head driver 15 to move the measurement heads 16 individually or in conjunction with each other in the X, Y, and Z directions, and to rotate each measurement heads 16 up and down, left and right about the corresponding eyeball rotation point of the subject E (see reference O in FIG. 5, for example.). Each measurement head 16 can be thereby moved to a position (posture) corresponding to the rotation of the corresponding subject eye E. The measurement head driver 15 adjusts the position of each measurement head 16 to diverge (divergence movement) or converge (convergent movement) the corresponding subject E. This allows the ophthalmologic apparatus 10 to test the divergence movement and the convergence movement and to examine at various test distances from a far-point test at a far-point distance to a near-point test at a near-point distance in binocular vision to measure various characteristics of both subject eyes E.

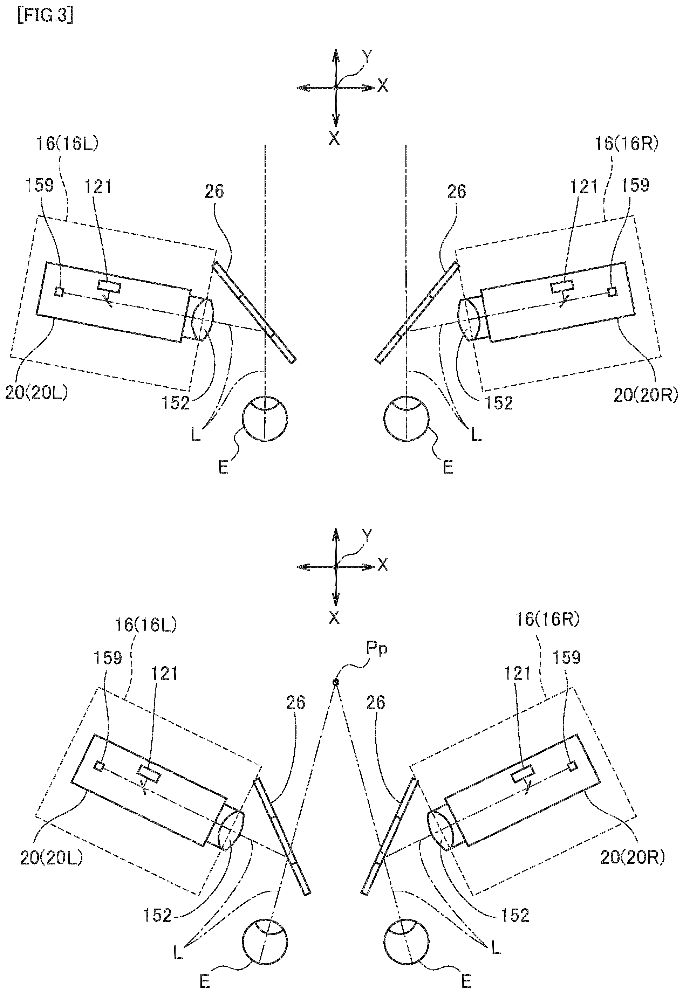

Each measurement head 16 includes a deflection member 26 (mirror) (individually referred to as left eye deflection member 26L and right eye deflection member 26R). Each measurement head 16 enables an after-described measurement optical system 20 (individually referred to as right eye measurement optical system 20R and left eye measurement optical system 20L) to acquire the information of the corresponding subject eye E through the deflection member 26. The measurement optical system 20 acquires the information of the corresponding subject eye E through the deflection member 26. As shown in FIG. 3, the ophthalmologic apparatus 10 can acquire the information of both subject eyes E simultaneously with the left and right both eyes of the subject open (binocular vision) when each deflection member 26 adjusts the position of each measurement head 16 to be at a position corresponding to each of the left and right subject eyes E of the subject S. The ophthalmologic apparatus 10 can also acquire the information of the subject eye E with the corresponding subject eye E viewed downward or upward when the X-axis rotation driver 25 changes the rotational posture of each measurement head 16 about the eyeball rotation axis in the X-axis direction. The ophthalmologic apparatus 10 can acquire the information of the subject eye E with the corresponding subject eye E viewed left and right when the Y-axis rotation driver 24 changes the rotational posture of each measurement head 16 about the eyeball rotation axis in the Y-axis direction.

Each measurement head 16 simultaneously changes the rotational posture in a left-right symmetrical manner about the eyeball rotation axis in the Y-axis direction of the corresponding subject eye E. This allows the direction of the measurement optical axis L of the optical system of the measurement optical system 20 change to match the visual line (visual line direction) that changes due to the divergence and the convergence of the corresponding subject eye E in binocular vision. The upper side of FIG. 3 illustrates that the rotational postures of both measurement optical systems 20 are adjusted so that the measurement optical axes L from both subject eyes E to the deflection members 26 are parallel. In this upper side of FIG. 3, the subject has a visual axis similar to that of the subject looking at infinity with binocular vision when each measurement optical system 20 presents the corresponding visual target image Sf, etc. to the subject eye E as described below. The lower side of FIG. 3 illustrates that the rotational postures of both measurement optical systems 20 are adjusted so that the measurement optical axes L from both subject eyes E to the deflection members 26 have their respective extended ends toward predetermined presentation positions Pp. In this lower state of FIG. 3, the subject has the visual axis similar to that of the subject looking at the presentation positions Pp with binocular vision when each measurement optical system 20 presents the visual target image Sf, etc. to the corresponding subject eye E. Thus, the ophthalmologic apparatus 10 simultaneously changes the rotational posture of each measurement head 16 left-right symmetrically to present the visual target image Sf at the position where the visual axes of both subject eyes E are changed so that both subject eyes E converge or diverge.

Therefore, each measurement optical system 20 functions as an acquisition optical system for acquiring the eye information of both subject eyes E. A pair of measurement head drivers 15, i.e., a pair of vertical driver 22 and horizontal driver 23 and a pair of Y-axis rotation driver 24 and X-axis rotation driver 25, function as an optical system changing mechanism that changes at least one of the position or the direction of each measurement optical system 20 as the acquisition optical system. The information of the rotational posture of each measurement optical system 20 (each measurement head 16) by a pair of measurement head drivers 15 is used as the optical posture information indicating the position and the orientation of the acquisition optical system. In addition, the alignment information acquired by a Z alignment system 110 and an XY alignment system 120 of each measurement optical system 20 is used as the subject eye position information indicating the position and the orientation of the subject eye E.

The support base 11 is provided with a controller 27 that totally controls each portion of the ophthalmologic apparatus 10. The controller 27 is housed in a control box (see FIG. 1). As shown in FIG. 4, the controller 27 is connected to the above-described each measurement optical system 20, each vertical driver 22, each horizontal driver 23, each Y-axis rotation driver 24, and each X-axis rotation driver 25 as the measurement head driver 15, as well as the examiner's controller 31, a subject's controller 32, and a memory 33. In the ophthalmologic apparatus 10, the electric power is supplied to the controller 27 from a commercial power source via a cable 28 (see FIGS. 1, 2), and the controller 27 supplies the electric power to the measurement head driver 15 and both measurement heads 16 (both measurement optical systems 20). The controller 27 can exchange information with the measurement head driver 15 and both measurement heads 16 (both measurement optical systems 20) to control the operations of those and appropriately acquire the information from them.

The examiner's controller 31 is used by an examiner to operate the ophthalmologic apparatus 10. The examiner's controller 31 is connected to the controller 27 through short-range wireless communication to enable communication with each other. The examiner's controller 31 is not limited to the configuration of Embodiment 1 as long as it is connected to the controller 27 via a wired communication path or a wireless communication path. The examiner's controller 31 of Embodiment 1 uses a portable terminal (information processing device) such as a tablet terminal or a smartphone. The examiner can hold the examiner's controller 31 or place the examiner's controller 31 on the optometry table 12 for the operation. The examiner can operate the examiner's controller 31 from any position regardless of the position of the subject or the position of the ophthalmologic apparatus 10, which increases the examiner's degree of freedom during measurement. The examiner's controller 31 is not limited to a portable terminal, but may be a notebook personal computer or a desktop personal computer, and may be fastened to the ophthalmologic apparatus 10. The examiner's controller 31 is not limited to the configuration of Embodiment 1.

The examiner's controller 31 includes a display 34 that is a liquid crystal monitor. The display 34 includes a display surface 34a (see e.g., FIG. 1) on which images, etc. are displayed, and a touch panel type input portion 34b superimposed thereon. The examiner's controller 31 appropriately displays on the display surface 34a an anterior eye image I (see e.g., FIG. 5) based on the image signal from an imaging element 159 provided in an anterior eye observation system 150, a measurement ring image, and a fundus image, etc., as described below under the control of the controller 27. The examiner's controller 31 is displayed with the input portion 34b under the control of the controller 27 and outputs to the controller 27 operation information such as an alignment instruction and a measurement instruction that are input to the input portion 34b.

The subject's controller 32 is used by a subject for response during acquiring the various ocular information of the subject E. The subject's controller 32 is an input device such as a keyboard, a mouse, and a joystick. The subject's controller 32 is connected to the controller 27 via a wired communication channel or a wireless communication channel.

The controller 27 develops a program stored in the connected memory 33 or a built-in internal memory 27a on a Random Access Memory (RAM), for example, to totally control the operation of the ophthalmologic apparatus 10 in response to the operation to the examiner's controller 31 and the subject's controller 32, as appropriate. In Embodiment 1, the internal memory 27a includes a RAM, and the memory 33 includes a Read Only Memory (ROM), an Electrically Erasable Programmable ROM (EEPROM). In addition to the above-mentioned configurations, the ophthalmologic apparatus 10 appropriately includes a printer that prints the measurement results in response to a measurement completion signal and an instruction from a measurer, and an output portion that outputs the measurement results to an external memory and a server.

Next, the optical configuration of the measurement optical system 20 as an example is described with reference to FIG. 5. As described above, the right eye measurement optical system 20R and the left eye measurement optical system 20L basically have the same configurations. In this case, they are simply described as the measurement optical system 20. Each measurement optical system 20 includes an eyesight test device that examines an eyesight while switching a visual target to be presented, a phoropter that acquires an appropriate corrected refractive power of the subject eye E while switching and positioning a correction lens, a refractometer and a wavefront sensor that measure a refractive power, a fundus camera that captures an image of a fundus, a tomography device that captures a tomographic image of a retina, a specular microscope that captures a corneal endothelium image, a keratometer that measures a shape of a cornea, and a tonometer that measures an intraocular pressure. The measurement optical system 20 includes these individually or in combination.

In the following description, "fundus conjugate position P" is meant to be a position that is substantially optically conjugate to the fundus Ef of the subject eye E in alignment, and the position that is optically conjugate to the fundus Ef of the subject eye E or its vicinity. The "pupil conjugate position Q" is meant to be a position that is substantially optically conjugate to the pupil of the subject eye E in alignment, and the position that is optically conjugate to the pupil of the subject eye E or its vicinity. As illustrated in FIG. 5, each measurement optical system 20 includes a Z alignment system 110, an XY alignment system 120, a keratometry measurement system 130, a visual target projection system 140, an anterior eye observation system 150, a refraction measurement projection system 160, and a refraction measurement light receiving system 170.

(Anterior eye observation system 150) The anterior eye observation system 150 captures a moving image and a still image of the anterior eye of the subject eye E for observing the anterior eye of the subject eye E. The anterior eye observation system 150 includes an anterior eye illumination light source 151, an objective lens 152, a first dichroic mirror 153, a half mirror 154, a first relay lens 155, a second relay lens 156, a second dichroic mirror 157, a first imaging lens 158, and an imaging element 159 (area sensor). The imaging element 159 has an imaging plane located at the pupil conjugate position Q in the optical system via the anterior eye observation system 150. The anterior eye illumination light source 151 illuminates the anterior eye of the subject eye E with illumination light (e.g., infrared light). In the anterior eye observation system 150, the light reflected by the anterior eye of the subject eye E passes through the objective lens 152, passes through the first dichroic mirror 153, passes through the half mirror 154, passes through the first relay lens 155 and the second relay lens 156, and passes through the second dichroic mirror 157. The light transmitted through the second dichroic mirror 157 is imaged on the imaging surface of the imaging element 159 by the first imaging lens 158. The imaging element 159 captures an image and outputs a signal at a predetermined rate. The output (video signal) of the imaging element 159 is input to the controller 27. The controller 27 displays an anterior eye image I based on the input video signal on the display 34 of the examiner's controller 31. The anterior eye image I is an infrared moving image, for example.

(Z alignment system 110) The Z alignment system 110 projects light (infrared light) for alignment in the optical axis direction (front and back direction, Z direction) of the anterior eye observation system 150 to the subject eye E. The Z alignment system 110 includes a Z alignment light source 111, a second imaging lens 112, and a line sensor 113. In the Z alignment system 110, the light is output from the Z alignment light source 111, is projected onto the cornea of the subject eye E, and the light is reflected by the cornea, and is imaged on the sensor surface of the line sensor 113 by the second imaging lens 112. In the Z alignment system 110, when the position of the corneal apex changes in the optical axis direction of the anterior eye observation system 150, the projection position of the light on the sensor surface of the line sensor 113 changes. The controller 27 determines the position of the corneal apex of the subject eye E based on the projection position of the light on the sensor surface of the line sensor 113, and controls the measurement head driver 15 to perform Z alignment based on this position.

(XY alignment system 120) The XY alignment system 120 irradiates light (infrared light) for alignment to the subject eye E in the directions orthogonal to the optical axis of the anterior eye observation system 150 (left and right direction (X direction) and up and down direction (Y direction)). The XY alignment system 120 shares the objective lens 152, the first dichroic mirror 153, and the half mirror 154 of the anterior eye observation system 150, and also includes an XY alignment light source 121. **In** the XY alignment system 120, the light output from the XY alignment light source 121 is reflected by the half mirror 154 to be projected through the anterior eye observation system 150 to the subject eye E. The reflected light from the cornea of the subject eye E is guided to the imaging element 159 through the anterior eye observation system 150, and the anterior eye image I includes an image based on this reflected light (bright spot image). The controller 27 displays the anterior eye image I including the bright spot image and the alignment mark on the display 34. When the X and Y directions are manually aligned, the user operates the movement of the optical system to guide the bright spot image within the alignment mark. When the X and Y directions are automatically aligned, the controller 27 controls the measurement head driver 15 to cancel the displacement of the bright spot image relative to the alignment mark.

(Keratometry measurement system 130) The keratometry measurement system 130 projects a ring shaped light beam of infrared light (kerato ring beam) for measuring the shape of the cornea of the subject eye E onto the cornea. The keratometry measurement system 130 includes a keratoplate 131 arranged between the objective lens 152 and the subject eye E. In the Keratometry measurement system 130, a kerato ring light source is provided on the back side (objective lens 152 side) of the keratoplate 131, although the illustration of the kerato ring light source is omitted. In the Keratometry measurement system 130, the light from the kerato ring light source illuminates the keratoplate 131, so that the ring shaped light beam is projected onto the cornea of the subject eye E. The reflected light from the cornea of the subject eye E (kerato ring image) is detected by the imaging element 159 together with the anterior eye image I. The controller 27 calculates the corneal shape parameter, which represents the shape of the cornea, with known calculation based on the kerato ring image. At this time, the controller 27 can appropriately display the image of the measurement pattern and the image of the reflected kerato ring light beam on the display 34 of the examiner's controller 31.

(Visual target projection system 140) The visual target projection system 140 presents a fixed visual target or a point visual target as a visual target to fix the visual line of the subject eye E during the objective examination, etc., or presents a subjective examination visual target for the subjective examination of the characteristics of the subject eye E (e.g., visual acuity value, corrected power (far-point power, near-point power)) under a specified presentation condition. The presentation condition includes a visual acuity value, a test distance, a test type, a visual target type, a magnification of a visual target, and a display mode of a visual target (e.g., shape, form, size, number, color, contrast of visual target). The visual target projection system 140 includes a light source 141, a collimator lens 142, a visual target chart 143, a third relay lens 144, a fourth relay lens 145, and a first reflection mirror 146, and also shares the objective lens 152 and first dichroic mirror 153 of the anterior eye observation system 150. In the visual target projection system 140, the light (visible light) output from the light source 141 is collimated into a parallel light beam by the collimator lens 142 to illuminate the visual target chart 143. The visual target chart 143 includes, for example, a transmissive liquid crystal panel and displays a visual target image Sf representing the visual target. The light source 141, the collimator lens 142, and the visual target chart 143 constitute the visual target unit 147, which can move integrally in the optical axis direction. The light transmitted through the visual target chart 143 passes through the third relay lens 144 and the fourth relay lens 145 in that order, is reflected by the first reflection mirror 146, passes through the after-described third dichroic mirror 168, and is reflected by the first dichroic mirror 153. The light reflected by the first dichroic mirror 153 passes through the objective lens 152 to be projected onto the fundus Ef.

In this visual target projection system 140, for the subjective examination, the visual target unit 147 is moved in the optical axis direction under the control of the controller 27 based on the results of the objective measurement. At this time, the controller 27 controls the display of the visual target chart 143 to display the visual target selected by the examiner or the controller 27 on the visual target chart 143. Thereby, the visual target projection system 140 presents the visual target selected by the examiner or the controller 27 to the subject at the predetermined presentation position Pp. The controller 27 receives the input of the subject's response to the visual target to further control and calculate the subjective examination value. This means that, for example, in eyesight measurement, the controller 27 selects and presents the next visual target based on the response to the Landolt ring, etc., and repeats this process to determine the eyesight value.

In this visual target projection system 140, when the visual target unit 147 moves along the optical axis, the presentation position Pp, which is the position of the visual target to be presented in the visual target unit 147, can be changed arbitrarily from the far-point test distance to the near-point test distance. Typically, the far-point test distance is 5.0 m and the near-point test distance is 30 cm or 50 cm, but in the ophthalmologic apparatus 10 of Embodiment 1, the visual target projection system 140 can arbitrarily change the test distance between 10 cm and 6.0 m. Here, the test distance of 1.0 m or more is defined as the far-point distance, and the test distance of less than 1.0 m is defined as the near-point distance. The test distance in the ophthalmologic apparatus 10 of Embodiment 1 is not the actual distance between the subject S (subject eye E) and the visual target, but the apparent distance created by the visual target projection system 140 as if the visual target were presented at a position at this distance.

Here, the visual target image Sf representing the visual target that the visual target chart 143 displays is not specially limited, as long as it is used for optometry in the subjective examination, for example. For example, the visual target image Sf suitably includes an eyesight test visual target, a red-green test visual target, an astigmatism test visual target, a binocular function test visual target, a Landolt ring, a Snellen visual target, and an E chart. The visual target image Sf can also be various visual targets including letters such as hiragana and katakana, a visual target such as a picture of an animal and a finger, and visual targets such as a specific figure, a landscape picture, and a landscape photograph for binocular function optometry such as a crossed visual target. Furthermore, the visual target image Sf may be a still image or a moving image. The visual target chart 143 includes a liquid crystal panel, which can display the visual target image Sf in a desired shape, form, and contrast, allowing for various and detailed optometry. The ophthalmologic apparatus 10 of Embodiment 1 also includes a visual target unit 147 in each measurement head 16 corresponding to the left and right subject eyes E. Therefore, the ophthalmologic apparatus 10 can display a visual target that gives parallax by a pair of visual target units 147 in accordance with a predetermined presentation position Pp, and can easily and preciously perform stereoscopic vision test with a natural visual axis orientation.

(Refraction measurement projection system 160, Refraction measurement light receiving system 170) The refraction measurement projection system 160 and the refraction measurement light receiving system 170 are the objective measurement optical systems used for the objective refraction measurement (refraction measurement). The refraction measurement projection system 160 projects a ring shaped light beam (infrared light) for the objective measurement onto the fundus Ef. The refraction measurement light receiving system 170 receives the return light of the ring shaped light beam from the subject eye E.

The refraction measurement projection system 160 includes a refraction measurement light source 161, a fifth relay lens 162, a conical prism 163, a field lens 164, a ring diaphragm 165, a hole prism 166, a rotary prism 167 and a third dichroic mirror 168. The refraction measurement projection system 160 shares the objective lens 152 and the first dichroic mirror 153 of the anterior eye observation system 150. The refraction measurement light source 161 may be a Superluminescent Diode (SLD) light source, which is a high brightness light source with an emission diameter less than a predetermined size. The refraction measurement light source 161 is movable in the optical axis direction and is disposed at the fundus conjugate position P. The ring diaphragm 165 includes its translucent portion disposed at the pupil conjugate position Q. The conical prism 163 should be positioned as close as possible to the pupil conjugate position Q.

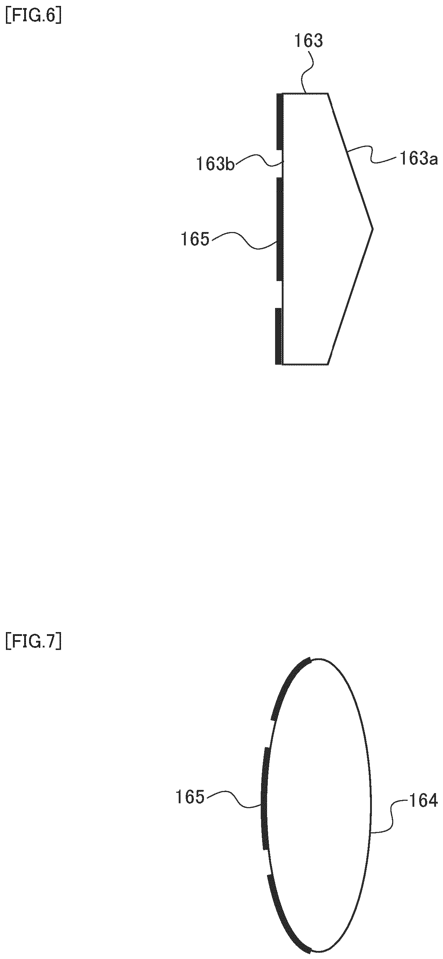

In the refraction measurement projection system 160, the light output from the refraction measurement light source 161 passes through the fifth relay lens 162 and enters the conical surface 163a (see FIG. 6) of the conical prism 163. The light incident on the conical surface 163a is deflected and emitted from the bottom surface 163b (see FIG. 6) of the conical prism 163. That emitted light passes through the field lens 164 and passes through the translucent portion formed in a ring shape on the ring diaphragm 165. The passed light (ring shaped light beam) is reflected by the reflection surface of the hole prism 166, passes through the rotary prism 167, and is reflected by the third dichroic mirror 168. The reflected light is reflected by the first dichroic mirror 153, passes through the objective lens 152, and is projected onto the subject eye E. The rotary prism 167 is used to average the light intensity distribution of the ring shaped light beam to the blood vessels and the disease sites in the fundus Ef and to reduce speckle noise caused by the light source.

Here, the field lens 164 may include, on its lens surface on the side of the subject eye E, the ring diaphragm 165 affixed thereto, as shown in FIG. 7. In this case, for example, a light shielding film is deposited on the lens surface of the field lens 164 so that the ring shaped translucent portion is formed. The refraction measurement projection system 160 may also include a configuration in which the field lens 164 is omitted. Furthermore, the conical prism 163 may include the ring diaphragm 165 affixed to the bottom surface 163b that emits the light that passes through the fifth relay lens 162 and enters the conical surface 163a, as shown in FIG. 6, for example. In this case, for example, a light shielding film is deposited on the bottom surface 163b of the conical prism 163 so that a ring shaped translucent portion is formed.

The ring diaphragm 165 may be provided on the side of the conical surface 163a of the conical prism 163. The ring diaphragm 165 may be a diaphragm with a translucent portion having a shape corresponding to a predetermined measurement pattern. Further, the ring diaphragm 165 may include a translucent portion formed at a position eccentric to the optical axis of the refraction measurement projection system 160. The ring diaphragm 165 may include two or more translucent portions.

The refraction measurement light receiving system 170 shares the objective lens 152, the first dichroic mirror 153, the second dichroic mirror 157, the first imaging lens 158, and the imaging element 159 of the anterior eye observation system 150. In addition, the refraction measurement light receiving system 170 shares the third dichroic mirror 168, the rotary prism 167, and the hole prism 166 of the refraction measurement projection system 160. The refraction measurement light receiving system 170 includes a sixth relay lens 171, a second reflection mirror 172, a seventh relay lens 173, a focusing lens 174, and a third reflection mirror 175. The focusing lens 174 is movable in the optical axis direction. The focusing lens 174 may be a known variable focusing lens that can change its focusing position under the control of the controller 27. The imaging element 159 is positioned in the optical system via the refraction measurement light receiving system 170 so that the imaging plane is positioned at the fundus conjugate position P. A diaphragm (not shown) is disposed between the hole prism 166 and the sixth relay lens 171 to limit the diameter of the light beam on the pupil. The translucent portion of this diaphragm is positioned at the pupil conjugate position.

In the refraction measurement light receiving system 170, the return light of the ring shaped light beam projected onto the fundus Ef by the refraction measurement projection system 160 passes through the objective lens 152 and is reflected by the first dichroic mirror 153 and the third dichroic mirror 168. The reflected return light passes through the rotary prism 167, passes through the hole in the hole prism 166, and passes through the sixth relay lens 171. The passed return light is reflected by the second reflection mirror 172 and passes through the seventh relay lens 173 and the focusing lens 174. The passed return light is reflected by the third reflection mirror 175, is reflected by the second dichroic mirror 157, and is imaged on the imaging surface of the imaging element 159 by the first imaging lens 158. The controller 27 calculates the refractive power value of the subject eye E with known calculation based on the output from the imaging element 159. For example, the refractive power value includes a spherical power, an astigmatism power, and an astigmatic axis angle.

The controller 27 moves the refraction measurement light source 161 and the focusing lens 174 in the optical axis direction based on the calculated refractive power value so that the fundus Ef, the refraction measurement light source 161, and the imaging plane of the imaging lens 159 are optically conjugated. Furthermore, the controller 27 moves the visual target unit 147 in the optical axis direction in conjunction with the movement of the refraction measurement light source 161 and the focusing lens 174. The visual target unit 147 including the light source 141, the collimator lens 142, and the visual target chart 143, the refraction measurement light source 161, and the focusing lens 174 may be moved in conjunction in the respective optical axis directions.

The ophthalmologic apparatus 10 acquires the eye information of the subject eye E using the measurement optical system 20 while automatically aligning (automatic alignment) under the control of the controller 27. In detail, the controller 27 calculates the movement amount (alignment information) that is a predetermined operation distance of the measurement optical system 20 to the subject eye E while aligning the measurement optical axis L of the measurement optical system 20 (its optical system) with the axis of the subject eye E based on the alignment information from the Z alignment system 110 and the XY alignment system 120. The operation distance is a default value also referred to as a working distance which is a distance between the measurement optical system 20 and the subject eye E for appropriately measuring the characteristics with the measurement optical system 20. The controller 27 drives the measurement head driver 15 according to the movement amount to move the measurement optical system 20 to the subject eye E, and to align the measurement optical system 20 (measurement head 16) in the XYZ directions with respect to the corresponding subject eye E. The controller 27 then appropriately drives the measurement optical system 20 to acquire the various eye information of the subject eye E. In the ophthalmologic apparatus 10, the examiner can also manually operate the examiner's controller 31 to align the measurement optical system 20 with the subject eye E, and drives the refraction measurement projection system 160 and the refraction measurement light receiving system 170 to acquire the various eye information of the subject E. In the ophthalmologic apparatus 10, when acquiring the various eye information of the subject eye E, the subject may respond with the operation of the subject's controller 32 to assist the acquisition of the various eye information of the subject eye E.

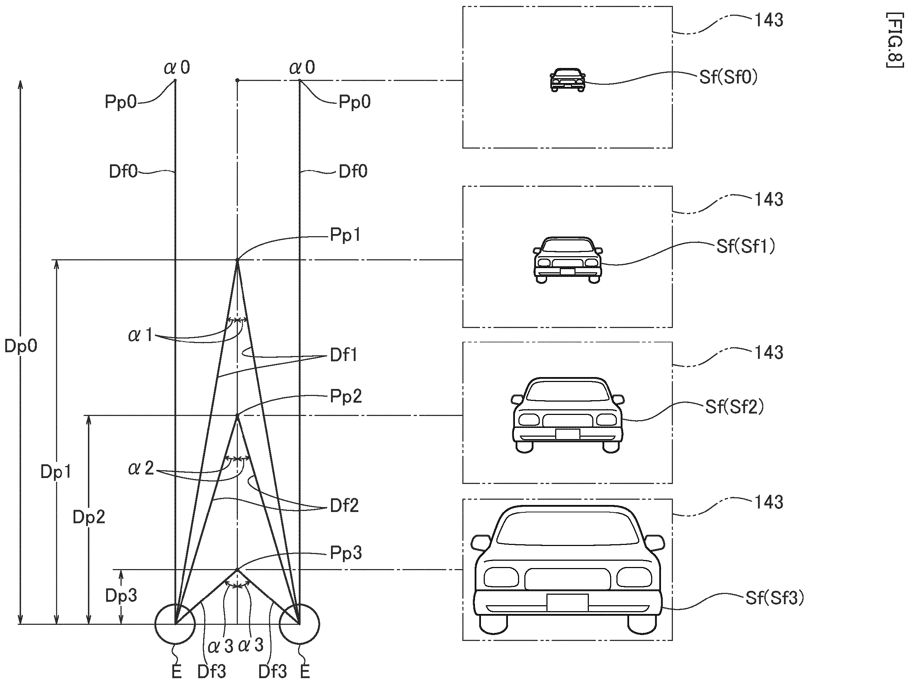

The ophthalmologic apparatus 10 can perform an adjustment force measurement process (adjustment force measurement mode). This adjustment force measurement process basically calculates the adjustment force (adjustment width) with the subtraction of the eye refractive power (FPA) at a far point from the eye refractive power (NPA) at a near point. The far point may be any set distance or may be infinity. The near point is a distance at which the subject (subject eye E) approaches the limit of what he/she can properly see. The adjustment force measurement process presents the visual target image Sf in binocular vision, and moves the presentation position Pp that presents the visual target image Sf from a predetermined far position to a predetermined near position (see FIG. 8) to measure the eye refractive power of each subject eye E at all times or at predetermined timing during the movement with the refraction measurement projection system 160 and the refraction measurement light receiving system 170.

The presentation position Pp indicates a position where the visual target image Sf is presented to measure the adjustment force, and as illustrated in FIG. 8, it can be moved from a predetermined far position to a predetermined near position to enable the measurement of the eye refraction force at the above-described far point and near point. The presentation position Pp in Embodiment 1 can be appropriately set, and as an example, it can be movable from infinity to 0.3 m. A predetermined far position and a predetermined near position (movable range) are appropriately set as long as they enable the measurement of the adjustment force, and are not limited to the configuration of Embodiment 1.

When the controller 27 displays the visual target image Sf on the visual target chart 143, the visual target image Sf is presented to each subject eye E using the visual target projection system 140. As illustrated on the right side of FIG. 8, the size of the visual target image Sf changes in accordance with the change in presentation position Pp, and the change in size is used to recognize that the presentation position Pp has changed. The visual target image Sf is smallest at infinity, gradually increases in size as it gets closer, and is largest at 0.3 m. This change is made by varying the size of the visual target image Sf displayed on the visual target chart 143. The magnification for this change (change rate) is set in accordance with the perspective when the change of the presentation position Pp is seen by naked eyes while an object shown in a chart as the visual target image Sf exists in the real world.

The visual target image Sf in Embodiment 1 is a moving image, and the above-described size change is continuous. This allows the subject to more naturally feel that the object shown by the visual target image Sf approaches (or move away). In addition, to more naturally recognize the change in presentation position Pp, the visual target image Sf in Embodiment 1 is based on what has been seen approaching or moving away in a real world, for example, a vehicle. The visual target image Sf of the example illustrated in FIG. 8 is only a vehicle, but may be displayed together with a simple background (e.g., straight road) to facilitate the understanding of perspective. The visual target image Sf is not limited to the configuration of Embodiment 1 as long as the size of the visual target image Sf changes for the recognition of the change in presentation position Pp. The visual target image Sf may be a plurality of frame-by-frame advance still images, and the mode of change, such as a magnification rate and the content of the drawing may be appropriately set, and are not limited to the configuration of Embodiment 1.

In the adjustment force measurement process, the controller 27 displays the visual target image Sf on the visual target chart 143 and changes the size of the visual target image Sf on the chart 143 according to the change in presentation position Pp. This allows the subject to recognize the visual target image Sf as approaching or moving away just by seeing the visual target image Sf with both subject eyes E, respectively.

In addition, the visual target image Sf may be provided with binocular parallax in accordance with the change in presentation position Pp. In this case, the visual target image Sf has no binocular parallax when it is the smallest in accordance with the presentation position Pp at infinity. When the presentation position Pp is closer than infinity, the visual target image Sf has binocular parallax as if the image displayed on the right eye measurement head 16R (right eye measuring optical system 20R) is seen obliquely forward right, and the image displayed on the left eye measurement head 16L (left eye measuring optical system 20L) is seen obliquely forward left. Specifically, the left and right visual target images Sf have binocular parallax in accordance with the visual target image Sf seen from the angle corresponding to the after-described rotation angle $\alpha$. The parallax given to the visual target image Sf is changed in accordance with the change in presentation position Pp, i.e., the change in rotation angle $\alpha$. In this way, when the visual target image Sf at an equal presentation position Pp is presented to each subject eye E, the subject can stereoscopically see the visual targe image Sf in accordance with the presentation position Pp, and can more clearly recognize an approaching image or a moving away image. At this time, the positions of both visual target images Sf presented to the right and left subject eyes E can be changed, respectively, according to the change in parallax and rotation angle $\alpha$. Such change in presentation position can make the stereoscopic vision more easily and appropriately.

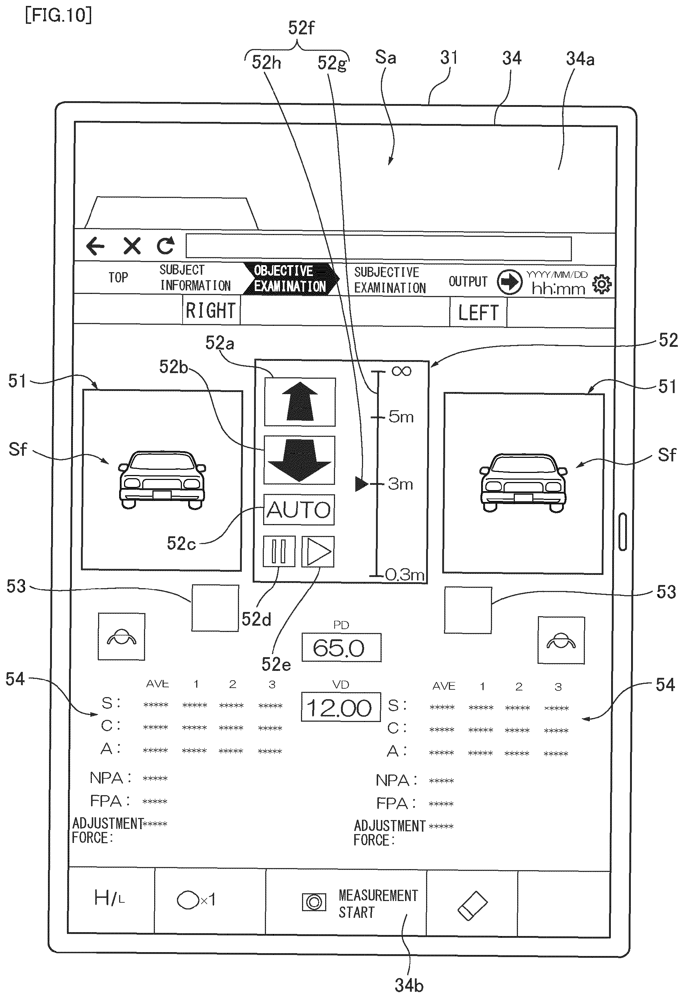

No parallax is required when the presentation position Pp is set to infinity, and the parallax is not very effective when the presentation position Pp is large to a certain extent, but is more effective the smaller the presentation position Pp is. Therefore, the range of the parallax can be appropriately set. As a result, even within the changeable range of the presentation position Pp, the visual target image Sf with parallax and the visual targe image Sf without parallax can be switched. This can improve the usability if the examiner can understand this, and for example, it can be displayed on the after-described adjustment force measurement screen Sa (see FIG. 10). Such display can be easily recognized without uncomfortable feeling by presenting the area (range) with parallax next to a scale 52g of the after-described operation display area 52.

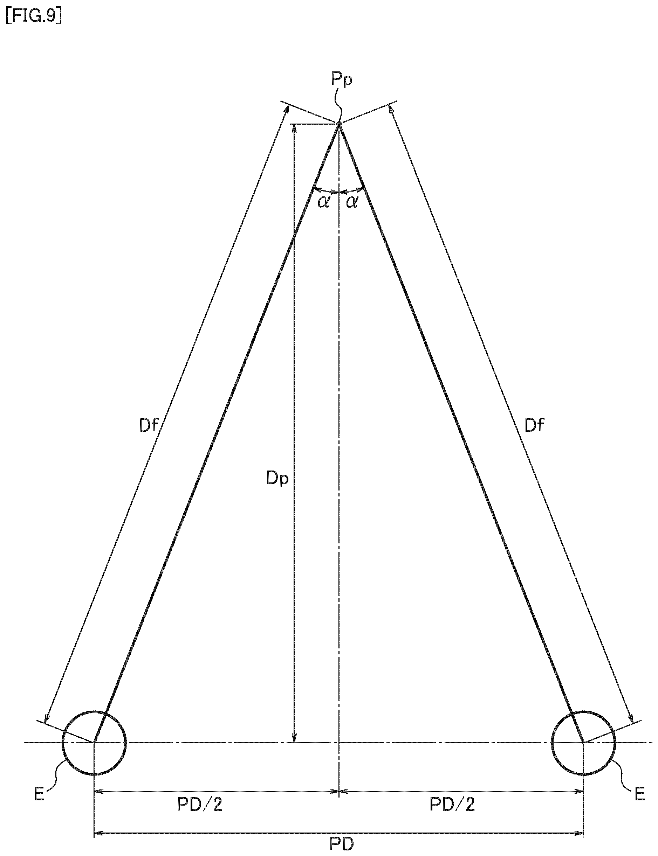

In the adjustment force measurement process, in addition to the change in size of visual target image Sf in accordance with the change in presentation position Pp as described above, the controller 27 totally changes the rotation angle α of the measurement optical system 20 (both measurement head 16) having the visual target projection system 140 and the focusing distance Df of the visual target image Sf in the visual target projection system 140. The rotation angle α is an angle of the direction of the measurement optical axis L of the visual target projection system 140 (measurement optical system 20) (direction from subject eye E to each deflection member 26) with respect to the state in which the visual target image Sf is displayed at infinity (parallel to each other). The direction of the measurement optical axis L can be adjusted by rotating both measurement heads 16 about the corresponding eyeball rotation point of the subject eye E as described above. Therefore, when the rotation angle α is 0 (zero) degree, the directions of both measurement optical axes L are parallel and the visual axis of the subject eye E is set to infinity. As illustrated in FIG. 9, the rotation angle α can be calculated from the pupil distance PD, which is the interval between both subject eyes E, and the presentation distance Dp, which is the interval from both subject eyes E to the presentation position Pp. In other words, the rotation angle α can be calculated by $\tan^{-1}(PD/2Dp)$. The pupil distance PD may be obtained from the anterior eye image I or the alignment position, or may use a general value.

In the adjustment force measurement process, the controller 27 displays the visual target image Sf on the visual target chart 143 and changes the rotation angle α of the visual target projection system 140 (measurement optical system 20 (both measurement heads 16)) that presents the visual targe image Sf to the subject eye E in accordance with the change in presentation position Pp. As a result, when both subject eyes see the visual target image Sf, the visual axis of each subject eye E converges or diverges according to the presentation position Pp, which makes the subject to recognize the visual target image Sf as approaching or moving away.

The focusing distance Df is the interval from each subject eye E to the presentation position Pp, which is a distance for the displayed visual target image Sf to be focused at the presentation position Pp. The focusing distance Df can be calculated from the presentation distance Dp, which is the distance from the center of both subject eyes E to the presentation position Pp, and the above-described rotation angle α. In other words, the focusing distance Df can be obtained with $Dp/\cos\alpha$.

In the adjustment force measurement process, the controller 27 displays the visual target image Sf on the visual target chart 143 and changes the focusing distance Df in the visual target projection system 140 (measurement optical system 20) that presents the visual targe image Sf to the subject eye E in accordance with the change in presentation position Pp. The focusing distance Df can be adjusted by moving the visual target unit 147 in the visual target projection system 140. This allows both subject eyes E to be in focus at the presentation position Pp when both subject eyes E see the visual target image Sf in focus, respectively, so that the subject can feel the visual target image Sf as approaching or as moving away.

When the controller 27 starts the adjustment force measurement process, as illustrated in FIG. 8, the controller 27 displays the smallest visual target image Sf0 on the visual target chart 143, sets the visual target projection system 140 to the infinity focusing distance Df0 and the measurement optical system 20 to the rotation angle α0 of 0 (zero) degree, and displays the visual targe image Sf0 at the infinity presentation position Pp0 (presentation distance Dp0). After that, the controller 27 displays the visual target image Sf1 larger than the visual target image Sf0 on the visual target chart 143, and displays the visual target image Sf at the presentation position Pp1, which is at the presentation distance Dp1, with the visual target projection system 140 as the focusing distance Df1 and the measurement optical system 20 as the rotation angle α1. Then, the controller 27 displays the visual target image Sf2 larger than the visual target image Sf1 on the visual target chart 143, and displays the visual targe image Sf at the presentation position Pp2 that is the presentation position Dp2 with the visual target projection system 140 as the focusing distance Df2 and the measurement optical system 20 as the rotation angle α. Thereafter, the controller 27 displays the visual target image Sf3 larger than the visual target image Sf2 on the visual target chart 143, and displays the visual targe image Sf at the presentation position Pp3 that is the presentation distance Dp3 with the visual target projection system 140 as the focusing distance Df3 and the measurement optical system 20 as the rotation angle α3.

Thus, the controller 27 totally changes the size of the visual target image Sf to be displayed on the visual target chart 143, the focusing distance Df of the visual target projection system 140, and the rotation angle α of the measurement optical system 20 according to the change in presentation position Pp. This makes the subject feel as if the visual target image Sf is approaching from a far point to a near point according to the change in presentation position Pp, makes it possible to show the visual target image Sf by converging both subject eyes E in a state extremely close to a natural state, and allows both subject eyes E to be adjusted appropriately. Similarly, the controller 27 changes the presentation position Pp from a near point to a far point. This makes the subject feel as if the visual target image Sf is moving away from a near point to a far point, makes it possible to show the visual target image Sf by diverging both subject eyes E in a state extremely close to a natural state, and allows both subject eyes E to be adjusted appropriately. In addition, when the visual target image Sf is provided with the binocular parallax in accordance with the change in presentation position Pp, the visual target image Sf can be presented with a three-dimensional impression, and both subject eyes E can converge or diverge and be adjusted more appropriately.

The controller 27 may automatically change the presenting position Pp or the examiner may instruct the change in presenting position Pp. In the adjustment force measurement process, as illustrated in FIG. 10, an adjustment force measurement screen Sa for an operation thereon can be displayed on the display surface 34a of the display 34. The adjustment force measurement screen Sa of Embodiment 1 has two visual target image display areas 51, an operation display area 52 provided therebetween, two limit operation buttons 53 provided therebelow, and two measurement value display areas 54 provided therebelow.

Both visual target image display areas 51 are areas where both visual target images Sf currently displayed are displayed, display the visual target image Sf displayed for the left eye on the right side of the front view of FIG. 10 and displays the visual target image Sf displayed for the right eye on the left side of the front view of FIG. 10. Both visual target image display areas 51 may display the anterior eye image I (see FIG. 5) acquired by the anterior eye observation system 150, respectively, along with the corresponding visual target image Sf. The anterior eye image I may be displayed at an area different from each visual target image display area 51.

The operation display area 52 is provided for the operation of changing the presentation position Pp in the adjustment force measurement process, and can be operated when a finger or a touch pen, for example, touches the operation display area 52 using the touch panel type input portion 34b superimposed on the display surface 34a. The operation display area 52 includes in the upper left corner a far movement symbol 52a for a far movement operation and therebelow a near movement symbol 52b for a near movement operation. The far movement symbol 52a and the near movement symbol 52b are for the examiner to manually change the presenting position Pp. The operation display area 52 includes below the near movement symbol 52b an automatic selection symbol 52c for automatically changing the presentation position Pp, in the lower left thereof a pause symbol 52d for pausing the automatic change of the presentation position Pp, and in the right thereof an execution symbol 52e for executing the automatic change of the presentation position Pp. Furthermore, the operation display area 52 includes in the right of each above-described symbol a presentation position indication symbol 52f for indicating the current presentation position Pp. This presentation position indication symbol 52f includes a scale 52g for indicating the presentation distance Dp from the subject eye E and a mark 52h for indicating the presentation position Pp on the scale 52g.

The limit operation button 53 is operated when the subject sees the blurred visual target image Sf moving toward the near side or when the subject is aware of diplopia (seeing two images), and is an operation area for detecting the presentation position Pp, which is the limit of the near side according to the subject's awareness. This limit operation button 53 may be operated by the subject or by the examiner based on the subject's response.

The measurement value display area 54 is an area where the various values measured in the adjustment force measurement process are displayed, and displays the measurement results of the left eye on the right side of the front view of FIG. 10, and displays the measurement results of the right eye on the left side of the front view of FIG. 10. The measurement value display area 54 in Embodiment 1 displays, from the top, a spherical power S as an eye refractive power, a cylindrical power C, an axial angle Ax, an eye refractive power NPA at a near point, an eye refractive power FPA at a far point, and an adjustment force (NPA-FPA). The measurement value display area 54 may display other measurement values together, and the order and the arrangement of the display may be appropriately set, and are not limited to the configuration of Embodiment 1.

The adjustment force measurement screen Sa may be of any other configuration (including function and design), may be displayed in any other location, and is not limited to the configuration of Embodiment 1 as long as it can be operated to perform the adjustment force measurement process and display the measurement results. Each operation described above may use a display different from the adjustment force measurement screen Sa, may be provided with an operation portion separate from the display 34, may be other configurations, and is not limited to the configuration of Embodiment 1.

The ophthalmologic apparatus 10 can measure the adjustment force of the subject eye E with the following adjustment force measurement process for example. First, the ophthalmologic apparatus 10 automatically aligns the measurement optical axis L of the optical system of the measurement optical system 20 with the visual axis (visual line direction) of the subject eye E while the moving image of the anterior eye image I acquired with the anterior eye observation system 150 is displayed on the display surface 34a. When the examiner's controller 31 or another operation portion is operated for measurement in the downward, upward, or left and right viewing state, the ophthalmologic apparatus 10 adjusts the direction of the measurement optical axis L of the optical system of the measurement optical system 20 according to the set direction (downward, upward, or left and right viewing). While the measurement optical axis L of the optical system of the measurement optical system 20 in each measurement head 16 is horizontal, when the ophthalmologic apparatus 10 measures the adjustment force of the subject eye E, such adjustment is not performed.

Next, the ophthalmologic apparatus 10 displays the smallest visual target image Sf0 on the visual target chart 143, the visual target projection system 140 is set to the focusing distance Df0 at infinity, the measurement optical system 20 is set to the rotation angle $\alpha 0$ of 0 (zero) degree, and displays the visual target image Sf at the presentation position Pp0 at infinity (presentation distance Dp0) (see FIG. 8). Thereafter, the ophthalmologic apparatus 10 moves the visual target unit 147 to a cloud state, sets the subject eye E to the adjustment pause state, and detects the measurement ring image using the refraction measurement projection system 160 and the refraction measurement light receiving system 170. The ophthalmologic apparatus 10 then calculates a spherical power, a cylindrical power, and an axial angle as the eye refractive power based on that measurement ring image using a well-known method.

Next, the ophthalmologic apparatus 10 places the visual target unit 147, the refraction measurement light source 161, and the focusing lens 174 at a diopter (D) position that conforms to the eye refractive power obtained in the above measurement, and fixes the visual target image Sf at infinity with the visual target projection system 140. The ophthalmologic apparatus 10 then calculates the eye refractive power (spherical power, cylinder power, and axial angle) with the refraction measurement projection system 160 and the refraction measurement light receiving system 170 in that state, and sets the value to the eye refractive power FPA at the far point. The eye refractive power FPA may be the eye refractive power measured with the optic image Sf presented at infinity instead of obtaining the eye refractive power with the forced cloud.

Next, the ophthalmologic apparatus 10 starts changing the presentation position Pp of the visual target image Sf to be presented at the near side, and measures the eye refractive power of each subject eye E with the refraction measurement projection system 160 and the refraction measurement light receiving system 170 always or predetermined timing during the change. At this time, the ophthalmologic apparatus 10 changes the presentation position Pp from infinity to near, and in accordance with the change, reduces the size of the visual target image Sf to be displayed on the visual target chart 143, shortens the focusing distance Df of the visual target projection system 140, and increases the rotation angle $\alpha$ of the measurement optical system 20. The ophthalmologic apparatus 10 then determines whether or not the limit operation button 53 is operated during the above operation, i.e., whether the subject is aware of the blurred visual target image Sf or diplopia. Alternatively, the ophthalmologic apparatus 10 may determine whether or not the presentation position Pp is changed to the set predetermined near position, i.e., the closest position in the set movement range (0.3 m in Embodiment 1). Then, the ophthalmologic apparatus 10 stops changing the presentation position Pp, and accordingly, stops changing the size of the visual target image Sf to be displayed on the visual target chart 143, changing the focusing distance Df of the visual target projection system 140, and changing the rotation angle $\alpha$ of the measurement optical system 20.

Then, the ophthalmologic apparatus 10 appropriately displays the eye refractive power NPA at a near point, the eye refractive power FPA at a far point, and the adjustment force (NPA-FPA) acquired by the eye refractive power measured while changing the presentation position Pp from far to near at each measured value display area 54 of the adjustment force measurement screen Sa on the display surface 34a (see FIG. 10). The eye refractive power NPA at a near point is acquired from the transition of the eye refractive power measured while changing the presentation position Pp. The eye refractive power NPA at a near point may be the eye refractive power immediately before the presentation position Pp that the subject is aware of the near limit. In addition, in Embodiment 1, the eye refractive power (spherical power, cylinder power, and axial angle) of both subject eyes E measured in each of the above steps is appropriately displayed at each measured value display area 54. When the reciprocal measurement is set, the ophthalmologic apparatus 10 changes the presentation position Pp that is changed to the closest position back to infinity to measure the eye refractive power, and to acquire the adjustment force with the eye refractive power.

Although this adjustment force measurement process is performed with binocular vision, some subjects have difficulty in binocular vision due to illness or any other reason. In such a case, the controller 27 drives only the measurement head 16 and the measurement optical system 20 corresponding to the subject eye, and performs each process in the same manner as described above in the monocular vison. The measurement in the monocular vision can be set with the examiner's controller 31 or other operation portion.

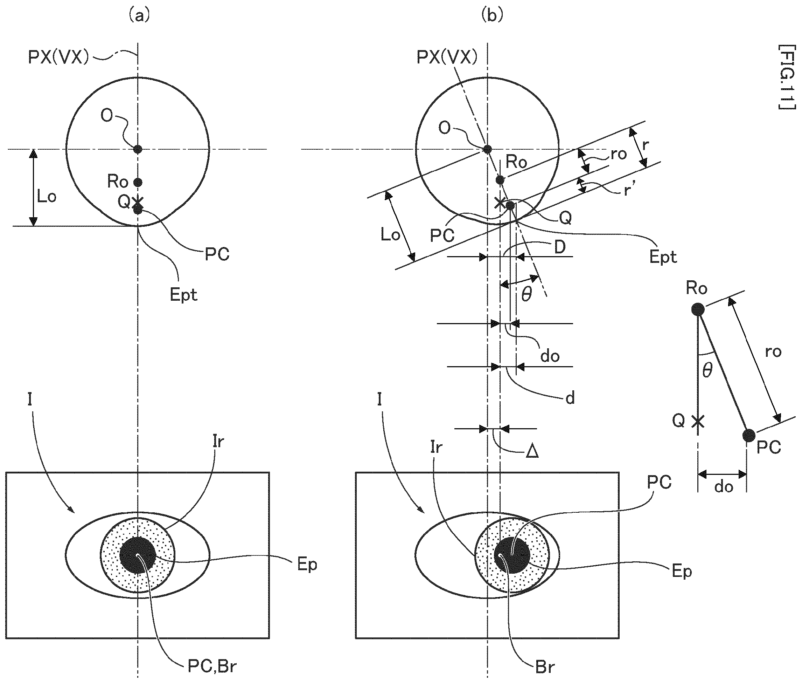

Next, the direction of the visual axis VX (see FIG. 12) when the subject eye E has heterotropia is explained with reference to FIG. 11. The left side of FIG. 11 illustrates the position of the bright spot Q of the XY alignment light source 121 in the subject eye E without heterotropia, and the right side of FIG 11 illustrates the position of the bright spot Q in the subject eye E with heterotropia. Here, the heterotropia is meant to be that the right and left eyes look toward different locations. For the subject eye E without the heterotropia (see left side of FIG. 11), when the right and left subject eyes visually fix on a common fixed visual target, the visual lines of the right and left subject eyes are directed toward a common location (fixed visual target) in the front. However, when the subject eye E has the heterotropia (see right side of FIG. 11), the visual line of the right or left subject eye is not directed to the fixed visual target, and therefore the visual lines of these subject eyes E is directed to different locations.

First, the subject eye E includes an axis connecting the fixed visual target (fixed viewpoint) and the center of the pupil (hereinafter referred to as pupil center PC) as a visual axis VX indicating where the subject eye E looks at. This visual axis VX can use, for example, the pupil axis PX that is the axis in the direction vertical to the cornea from a pupil center PC perpendicular to the cornea in the subject eye E. The pupil axis PX (visual axis VX) in the subject eye E can be known from the position of the bright spot image Br (hereinafter also referred to as corneal reflection Br) based on the XY alignment light source 121. The pupil axis PX in the subject eye E can be known from the position of the pupil center PC. As illustrated in the left side of FIG. 11, the subject eye E without the heterotropia has the center of the pupil image Ep in the anterior eye image I, i.e., the pupil center PC and the corneal reflection Br at the same position. In contrast, as illustrated in the right side of FIG. 11, the subject eye E with the heterotropia has the position of the corneal reflection Br shifted from the pupil center PC. Therefore, the ophthalmologic apparatus 10 of the present embodiment determines the shift amount (distance d0) between the pupil center PC and the position of the corneal reflection Br, and determines the angle θ between the pupil axis PX and the optical axis of the measurement optical system 20 from the shift amount.

As an example, the ophthalmologic apparatus 10 determines the shift between the pupil axis PX (visual axis VX) and the optical axis based on the Hirschberg method. The Hirschberg method is an objective quantitative examination with which an examiner at a position coaxial to the light source measures the relationship between the pupil center PC and the position of the corneal reflection Br after the examiner instructs to the subject to visually fix the light source placed at 33 cm from the subject eye E. Here, the XY alignment light source 121 that leads the corneal reflection Br and the imaging element 159 of the anterior eye observation system 150 are on the same optical axis, so that the ophthalmologic apparatus 10 determines the pupil center PC from the anterior eye image I captured by the imaging element 159 and also determines the position of the corneal reflection Br in the anterior eye image I to perform the objective quantitative examination of the heterotropia in accordance with the Hirschberg method.

Specifically, the controller 27 displays the fixed visual target on the visual target chart 143 of the visual target projection system 140 of the ophthalmologic apparatus 10. Next, the controller 27 rotates both measurement heads 16 about the eyeball rotation axis of the corresponding subject eye E with the measurement head driver 15 so that the subject visually fixes on the fixed visual target displayed at the predetermined presentation position Pp (see FIG. 9) located at 33 cm in front of the subject eye E. Each subject eye E thereby converges to gaze the fixed visual target. In this state, the controller 27 determines the pupil center PC of the pupil image Ep in the anterior eye image I and the position of the corneal reflection Br, respectively. In the ophthalmologic apparatus 10 of Embodiment 1, the XY alignment light source 121 is infrared light, which can simply and accurately acquire the position of the corneal reflection Br based on the reflected light from the XY alignment light source 121 by extracting only a signal in the infrared light region from the output signal of the imaging element 159. The controller 27 then acquires the shift amount of the position of the corneal reflection Br to the position of the pupil center PC (hereinafter referred to as distance d0) based on the coordinates of the pupil center PC and the position coordinates of the corneal reflection Br.

The angle θ between the pupil axis PX and the optical axis of the measurement optical system 20 can generally be calculated based on the following equation (1) using the curvature radius of the cornea r (i.e., distance from the center of corneal curvature Ro to corneal apex Ept) and the distance d between the position of the corneal apex Ept and the position of corneal reflection Br. The distance d can be obtained based on the anterior eye image I.

$$\sin\theta = d/r \quad \cdot\cdot\cdot \quad (1)$$

The angle θ may be acquired based on the following equation (2) using the distance d0 (shift amount) and the distance r0 from the center of corneal curvature Ro to the pupil center PC. This allows more efficient calculation of the angle θ based on the anterior eye image I.

$$\sin\theta = d0/r0 \quad \cdot\cdot\cdot \quad (2)$$

Here, for example, the distance r0 is acquired by subtracting the distance r' between the corneal apex Ept and the pupil center PC from the curvature radius r of the cornea. The curvature radius r can be a general average value of 7.7 mm, for example, and the distance r' can be a general average value of 3.6 mm, for example. In this case, the distance r0 = (7.7 - 3.6) mm = 4.1 mm. The respective positions of the pupil center PC and corneal reflection Br are easily affected by the refraction effect of the cornea, and the distance r0 includes an individual difference. Therefore, the distance d0 and the distance r0 may be collected for the pupil axis PX of the subject eye E without the heterotropia as shown on the left side of FIG. 11 and for the pupil axis PX of the subject eye E facing various other directions, and the distance r0 may be optimized based on these simultaneous equations. Alternatively, the distance r0 may be optimized from the actual measurement value of the curvature radius r of the cornea acquired by keratometry measurement.

Instead of the calculation using the above (1) and (2), the angle θ can also be calculated using the following equation (3). In the following equation (3), L0 is the distance from the corneal apex Ept to the eyeball rotation point O and D is the distance between the position of the corneal apex Ept and the position of the eyeball rotation point O. The distance L0 from the corneal apex Ept to eyeball rotation point O may be a predetermined value (e.g., average value of 13 mm). Alternatively, when the actual distance is known in measurement by another device, this value may be input. The curvature radius r of the cornea can be a measured value acquired by the keratometry measurement. The curvature radius r of the cornea may use the average value (7.7 mm) as the initial value. Also in this case, the distance from the pupil center PC to the eyeball rotation point O may be used instead of the distance L0, and the distance between the position of the pupil center PC and the position of the eyeball rotation point O in the anterior eye image I may be used instead of the distance D for the calculation.

$$\sin\theta = D/L0 \quad \cdot \quad \cdot \quad \cdot \quad (3)$$

A further method of calculating the different angle θ can be, for example, a displacement Δ of the corneal reflection Br (see right side of FIG. 11). The displacement Δ can be expressed as the amount of displacement of the corneal reflection Br from the eyeball rotation point O by acquiring each value in the left side state of FIG. 11 with only the subject eye E for which the displacement was detected being fixed to the fixed visual target.

When the distance from the corneal apex Ept to the eyeball rotation point O is L0 and the curvature radius of the cornea is r, the displacement Δ of the corneal reflection Br illustrated in the right side of FIG. 11 can be expressed as the following equation (4). In this case, the distance L0 from the corneal apex to the eyeball rotation point O may be a predetermined value (e.g., average value of 13 mm). Alternatively, when the actual distance is known in the measurement by another device, this value may be input. The curvature radius r of the cornea can be a measured value or an average value (7.7 mm) acquired by the keratometry measurement.

$$\Delta = (L0-r)\cdot\sin\theta \ \dots \ (4)$$

The controller 27 then calculates the angle θ between the pupil axis PX (visual axis VX) and the optical axis as described above. This angle θ is the subject eye visual line information indicating the direction of the visual line (visual axis VX) of the subject eye E.

In the ophthalmologic apparatus 10, the controller 27 creates a positional relationship image Ip and displays it on the display 34 of the examiner's controller 31. As illustrated in FIG 12, the positional relationship image Ip shows the positional relationship of the measurement optical system 20 (both measurement head 16) with respect to both subject eyes E when the measurement optical system 20 measures the adjustment force of both subject eyes E, for example. The positional relationship image Ip in this example includes at least a pair of measurement optical system patterns Dm that imitates a pair of measurement optical systems 20, and a pair of subject eye patterns De that imitates a pair of subject eyes E. The controller 27 creates the positional relationship image Ip based on the optical posture information from each measurement head driver 15 as the optical system change mechanism and the subject eye positional information from each measurement optical system 20 as the acquisition optical system. In other words, the controller 27 finds the position and the orientation of each measurement optical system 20 to each subject eye E based on the optical posture information and the subject eye position information. The controller 27 sets the positions of a pair of subject eye patterns De based on the position and the orientation of each measurement optical system 20 to each subject eye E and the positions and the orientations of a pair of measurement optical system patterns Dm to the position and the orientation of each measurement optical system 20 to each subject eye E to create the positional relationship image Ip. Each measurement optical system pattern Dm displays the measurement optical axis, which makes it easy to find the orientation. The controller 27 then displays the created positional relationship image Ip on the display 34 of the examiner's controller 31.

The positional relationship image IP in FIG. 12 includes, in addition to each subject eye pattern De and each measurement optical system pattern Dm, a pair of measurement head patterns Dh that imitates a pair of measurement heads 16, and a pair of deflection member patterns Dd that imitates a pair of deflection members 26. Each measurement head pattern Dh and each deflection member pattern Dd can make it easier to understand how each subject eye E actually faces each measurement head 16. The positions and the orientations of each measurement head pattern Dh and each deflection member pattern Dd can be set the position and the orientation based on the optical orientation information. For this reason, in the positional relationship image Ip, the measurement optical axis L is depicted as extending from both subject eyes E, reflecting off each deflection member pattern Dd, and reaching each measurement optical system pattern Dm.

The positional relationship image Ip in the example FIG. 12 includes a various value display area Vd. In this example, the various value display area Vd includes a presentation distance information I1 indicating the presentation distance Dp, a rotation angle information I2 indicating the rotation angle α, a focusing distance information I3 indicating the focusing distance Df, a pupil distance information I4 indicating the pupil distance PD, and a proximity degree information I5 indicating the proximity degree. The presentation distance information I1, the rotation angle information I2, and the focusing distance information I3 indicate the presentation distance Dp, the rotation angle $\alpha$, and the focusing distance Df acquired as described above, and are changed according to the change in each measurement optical system 20 (both measurement head 16) in accordance with the presentation position Pp. The pupil distance information I4 shows the pupil distance PD acquired as described above, which is a constant value for the same subject. The proximity degree information I5 indicates the interval Pi from the closest area to the corresponding subject eye E in each measurement optical system 20. In Embodiment 1, the closest area is the tip of the deflection member 26. The position (tip) of the deflection member 26 in each measurement optical system 20 is known in advance, so that the proximity degree information I5 (interval Pi) can be acquired from the optical posture information of each measurement optical system 20 and the subject eye position information of the corresponding subject eye E. The closest area may be appropriately set and is not limited to the configuration of Embodiment 1.

Although the position of the deflection member 26 (tip of deflection member) in each measurement optical system 20 is known in advance, deviations from the set position in each measurement head driver 15 may occur due to assembly, for example. Therefore, the ophthalmologic apparatus 10 corrects the deviation of the reference position for the movement by each measurement head driver 15 and the position of the deflection member 26 (tip) in each measurement optical system 20 at the time of shipment to acquire more accurate proximity degree information I5 (interval Pi). When the acquired proximity degree information I5 becomes smaller than a predetermined value (excessively close), the ophthalmologic apparatus 10 may blink the proximity degree information I5 or change the color of the proximity degree information I5 to emphasize the proximity degree information I5, change the color or the display mode of the display 34, or generate for example sounds for attention. This predetermined value may be set in consideration of the general positional relationship (distance) of an area that can be located in front of the subject eye E around the subject eye such as a cheek bone and an orbital border with respect to the subject eye E. This allows a subject having a high cheek bone or a high orbital border to appropriately avoid the contact by the deflection member 26 (tip), for example.

The positional relationship image Ip in the example of FIG. 12 displays the visual axis VX along with each subject eye pattern De to indicate the orientation of each subject eye pattern De. Here, the visual axis VX basically aligns with the measurement optical axis L of each measurement optical system 20 (measurement optical system pattern Dm). However, for the subject eye E with the heterotropia, the measurement optical axis L and the visual axis VX have an angle θ determined as described above (see visual axis VX indicated by dashed line from subject eye E on left side of FIG. 12). This makes it possible to easily understand the direction of the visual axis VX of the subject eye E with the heterotropia by displaying the visual axis VX along with each subject eye pattern De in the positional relationship image Ip.

In FIG. 12, the positional relationship image Ip is displayed over the entire area of the display 34 (i.e., full screen display), but it can also be one of various displays (partial display) as in FIG. 10, and is not limited to the example in FIG. 12. In this case, the positional relationship image IP can be switchable between the full screen display and the partial display to improve its usability.

In the positional relationship image Ip in the example of FIG. 12, each measurement optical system pattern Dm has a deflection member pattern Dd corresponding to the deflection member 26 in accordance with the actual optical path in each measurement optical system 20. However, the understanding of the positional relationship of the visual target image Sf to a pair of subject eyes E may be improved. For this reason, the positional relationship image Ip may show the visual target image Sf instead of each measurement optical system pattern Dm, as illustrated in FIG. 13. In this case, as illustrated by the single-dotted line, each measurement optical system pattern Dm may be displayed together.

Next, the problem of the ophthalmologic apparatus technology is explained. In the above-described conventional ophthalmologic apparatus, the subject places his/her face against the head portion in the appropriate posture of the subject, and the head portion is provided with the measurement optical system. Such a configuration of the conventional ophthalmologic apparatus makes it difficult to visually see the subject eye and the measurement optical system. Therefore, the examiner cannot objectively determine the positional relationship between the subject eye and the acquisition optical system, which makes it difficult to confirm the condition of the test (optometry) of the visual function of the subject eye.

In contrast, in the ophthalmologic apparatus 10 of the present disclosure, the controller 27 creates the positional relationship image Ip having a pair of measurement optical system patterns Dm that imitates a pair of measurement optical systems 20 and a pair of subject eye patterns De that imitates a pair of subject eyes E to display the positional relationship image Ip on the display 34 of the examiner's controller 31. Therefore, the ophthalmologic apparatus 10 allows the examiner to intuitively understand the positional relationship of each measurement optical system 20 with respect to each subject eye E through the positional relationship image Ip, and to easily confirm the condition of the test (optometry) of the visual function of the subject eye E. In particular, the ophthalmologic apparatus 10 displays the measurement optical axis L along with each measurement optical system pattern Dm in the positional relationship image Ip, which makes it more appropriately to confirm the condition of the test (optometry) of the visual function of the subject eye E.

The positional relationship image Ip includes a pair of measurement head patterns Dh that imitates a pair of measurement heads 16 and a pair of deflection member patterns Dd that imitates a pair of deflection members 26. Therefore, the actual positional relationship between each measurement head 16 and each subject eye E can be recognized in the positional relationship image Ip. This allows the examiner to understand how each subject eye E faces each measurement head 16 and to confirm the positional relationship of both subject eyes E with respect to the respective measuring optical systems 20.

Furthermore, the ophthalmologic apparatus 10 also displays the various value display area Vd in the positional relationship image Ip. Therefore, the ophthalmologic apparatus 10 allows the examiner to understand the positional relationship of each measurement optical system 20 with respect to each subject eye E even with various numerical values and to more appropriately confirm the condition of test (optometry) of the subject eye E. In particular, the ophthalmologic apparatus 10 displays the proximity degree information I5 as the various value display area Vd, which makes it possible to control each measurement optical system 20 (deflection member 26) from contacting each subject eye E even when the face of the subject E is put to each measurement head 16.

The ophthalmologic apparatus 10 of Embodiment 1 of the ophthalmologic apparatus according to the present disclosure has the following effects. The ophthalmologic apparatus 10 includes the measurement optical system 20 as a pair of acquisition optical systems for acquiring eye information of both subject eyes E and the measurement head driver 15 as the optical system change mechanism that changes at least one of the position or the orientation of the measurement optical systems. In the ophthalmologic apparatus 10, the controller 27 creates the positional relationship image IP showing the positional relationship of a pair of measurement optical systems 20 with respect to a pair of subject eyes E based on the optical posture information indicating the position and the orientation of a pair of measurement optical systems 20 and the subject eye positional information indicating the position of the subject eye E to display the positional relationship image Ip on the display 34. Therefore, the ophthalmologic apparatus 10 allows the examiner to intuitively understand the positional relationship of each measurement optical system 20 with respect to each subject eye E through the positional relationship image Ip and to easily confirm the condition of the test (optometry) of the visual function of the subject eye E.

In the ophthalmologic apparatus 10, the positional relationship image IP includes the information of the measurement optical axis L in a pair of measurement optical systems 20. Therefore, the ophthalmologic apparatus 10 allows the examiner to more appropriately confirm the condition of the test (optometry) of the visual function of the subject eye E.

Furthermore, the ophthalmologic apparatus 10 uses the subject eye positional information as the alignment information in the measurement optical system 20. The ophthalmologic apparatus 10 uses the alignment information that each measurement optical system 20 acquires for the measurement, so that the ophthalmologic apparatus 10 can acquire the subject eye positional information with a simple configuration.

In the ophthalmologic apparatus 10, the positional relationship image Ip has the proximity degree information I5 indicating the proximity degree from a pair of measurement optical systems 20 to the corresponding subject eye E. Therefore, the ophthalmologic apparatus 10 can control each measurement optical system 20 (deflection member 26) from contacting each subject eye E even when the face is put to each measurement head 16.

The ophthalmologic apparatus 10 shows the positional relationship image Ip with a positional relationship that looks down a pair of subject eyes E and a pair of measurement optical systems 2 from above, which allows the examiner to understand how the acquisition optical system is displaced in the left and right direction relative to the subject eye E. Therefore, the ophthalmologic apparatus 10 allows the examiner to easily understand how the subject eye E diverges (divergent motion) or converges (convergent motion) with respect to the visual target image Sf shown by a pair of measurement optical systems 20. In the ophthalmologic apparatus 10, the positional relationship image IP has the positional relationship that looks down a pair of subject eyes E and a pair of measurement optical systems 20 from above. Therefore, the ophthalmologic apparatus 10 allows the subject to easily understand how the subject eye E diverges (divergent motion) or converges (convergence motion) with respect to the visual target image Sf shown by a pair of measurement optical systems 20.

In the ophthalmologic apparatus 10, the positional relationship image IP has at least one of the pupil distance information I4 indicating the interval between a pair of subject eyes E, the focusing distance information I3 indicating the focusing distance of the visual target image Sf presented by a pair of acquisition optical systems (measurement optical system 20), and the rotation angle information I2 indicating the rotation angle $\alpha$ of a pair of acquisition optical systems (measurement optical system 20). Therefore, the ophthalmologic apparatus 10 allows the examiner to understand the positional relationship of each measurement optical system 20 with respect to each subject eye E even with various numerical values, and to more appropriately confirm the condition of the test (optometry) of the visual function of the subject eye E.

Accordingly, the ophthalmologic apparatus 10 as an example of the ophthalmologic apparatus of the present disclosure allows the examiner to objectively understand the positional relationship between the subject eye E and the measurement optical system 20 as the acquisition optical system even when the subject places his/her face against the measurement head 16.

[Modified Example]

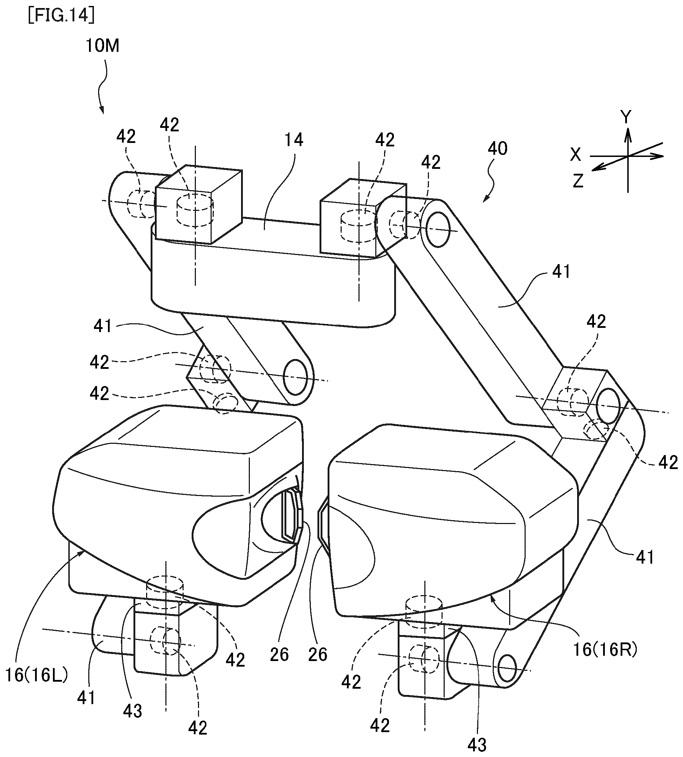

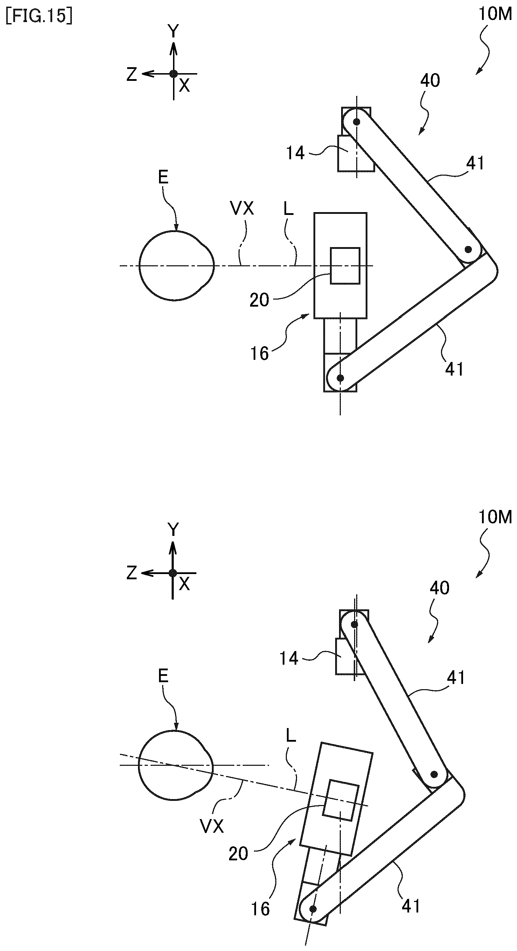

The above-described ophthalmologic apparatus 10 adjusts the positions of both measurement heads 16 to diverge (divergence movement) or converge (convergence movement) the corresponding subject eye E. In addition to this, the measurement head 16 can rotate in the up and down direction (rotation direction about X direction) about the eyeball rotation axis of the subject eye E in the X-axis direction. This configuration that rotates both measurement heads 16 in the up and down direction is described with reference to FIGS. 14 to 17 as an ophthalmologic apparatus 10M of the modified example of Embodiment 1. In FIGS. 14, 15, the configuration for supporting the support arms 14, etc., is omitted to facilitate the understanding of the configuration, and only the configuration in which both measurement heads 16 can be moved by a measurement head driver 40 having a configuration different from the ophthalmologic apparatus 10 is illustrated.

The ophthalmologic apparatus 10M of the modified example is basically similar in configuration to the above-described ophthalmologic apparatus 10, and includes both measurement heads 16 in the same configuration as the above-described ophthalmologic apparatus 10 as illustrated in FIGS. 14, 15. The ophthalmologic apparatus 10M includes a different configuration of the measurement head driver 40 that moves both measurement heads 16 from the measurement head driver 15 of the above-described ophthalmologic apparatus 10. The measurement head driver 40 is provided in the support arm 14 having the same configuration as that of the above-described ophthalmologic apparatus 10. The measurement head driver 40 three-dimensionally drives both measurement heads 16 in the XYZ directions to arrange both measurement heads 16 (measurement optical system 20 provided therein) at desired positions (e.g., test distance, height, right and left positions) and desired postures (inclination) with respect to the subject eye E. The measurement head driver 40 is paired in accordance with each of the paired measurement heads 16, and its configuration is symmetrical in the left and right direction. Thus, the paired measurement head drivers 40 are hereinafter described simply as the measurement head driver 40.

The measurement head driver 40 includes two arms 41, six rotation drivers 42, and a support portion 43. One of the arms 41 is attached to the top portion of the support arm 14 via two rotation drivers 42. The measurement head driver 40 may be mounted on the optometry table 12 (see FIG. 1) or in any other position, and is not limited to the configuration of the modified example. Each rotation driver 42 is paired at the positions of both ends of each support arm 41 and can rotate about two orthogonal axis lines. Each rotation driver 42 includes a mechanism that holds the shaft body by a bearing, for example, and a motor that generates the driving force for each. The support portion 43 supports both measurement heads 16 via the two rotation drivers 42.

In this ophthalmologic apparatus 10M, the controller 27 (see e.g., FIG. 1) having a configuration similar to the above-described ophthalmologic apparatus 10 obtains the current position information and the posture information of both measurement heads 16 (measurement optical system 20 (measurement optical axis L)) based on various information such as an acceleration, an angular velocity, an azimuth, position coordinates, a distance from the subject S, an angle of the subject S's face and posture information to control the driving of the measurement head driver 40 based on the position information and the posture information. The controller 27 controls the measurement head driver 40 based on the test distance and the visual recognition target that are specified by the examiner's controller 31. Thus, the controller 27 can change the position and the posture of the measurement optical system 20 (measurement optical axis L) of both measurement heads 16 with respect to the subject eye E as desired, and can arrange both measurement heads at predetermined positions in the XYZ directions (left and right, up and down, and front and back) where the visual recognition targets are arranged in a daily life.

The measurement head driver 40 includes a robot arm with 6 axes in Embodiment 2 or may include a robot arm with 5 or 7 or more axes. The measurement head driver 40 includes a multi-joint type (vertically articulated) robot arm that is one of the serial link type, but it is not limited to this configuration and may include a horizontally articulated robot arm, a coordinate axis type (e.g., rectangular coordinates, cylindrical coordinates, curved coordinates) robot arm, and a parallel link type robot arm. Furthermore, the measurement head driver 40 may be manually moved as long as it can move both measurement heads 16 in three dimensions to position them in a desired position and a desired posture. When the measurement head driver 40 is moved manually, the controller 27 can acquire the three-dimensional position of both measurement heads 16 moved by the measurement head driver 40 with a sensor for detecting the three-dimensional position of the measurement head 16.

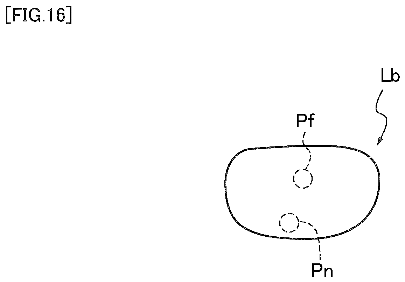

This ophthalmologic apparatus 10M can basically perform the examination similar to that of the above-described ophthalmologic apparatus 10. In addition to this, the ophthalmologic apparatus 10M allows the subject S to confirm the optical settings and the viewing of the bifocal lens Lb such as a double focus lens or a progressive lens. As illustrated in FIG. 16, the bifocal lens Lb includes a far-point portion and a near-point portion and includes a set far-point position Pf that is the refractive power measurement position of the far-point portion and a near-point position Pn that is the refractive power measurement position of the near-point portion. The ophthalmologic apparatus 10M can perform a far-point test on the subject eye E in the direction corresponding to the far-point position Pf and a near-point test in the direction corresponding to the near-point position Pn.

First, as illustrated in the upper side of FIG 15, in the ophthalmologic apparatus 10M, both measurement heads 16 are arranged in a position and a posture to face the subject eye E such that the measurement optical axis L is positioned at the far-point position Pf of the bifocal lens Lb (see FIG. 16). In this posture, the ophthalmologic apparatus 10M performs the objective examination by presenting an the visual target at the far-point test distance by the visual target projection system 140 of the measurement optical system 20. The ophthalmologic apparatus 10M thereby acquires the eye information of the subject eye E in the direction of the far-point position Pf.

Next, as illustrated in the lower side of FIG. 15, in the ophthalmologic apparatus 10M, both measurement heads 16 are arranged in a position and a posture to face the subject eye E such that the measurement optical axis L is positioned at the near-point position Pn of the bifocal lens Lb (see FIG. 16). In this posture, the ophthalmologic apparatus 10M performs the objective examination by presenting the visual target at the near-point test distance by the visual target projection system 140 of the measurement optical system 20. The ophthalmologic apparatus 10M thereby acquires the eye information of the subject eye E in the direction of the near-point position Pn. The ophthalmologic apparatus 10M may perform the near-pint test with both measurement heads 16 in the position illustrated in the upper side of FIG. 15.

Next, the ophthalmologic apparatus 10M confirms the visibility, etc., at the far-point position Pf and the near position Pn for the subject S wearing the bifocal lens Lb prescribed according to the above-described eye information. In other words, in the ophthalmologic apparatus 10M, with the subject S wearing the bifocal lens Lb as illustrated in the upper side of FIG 15, both measurement heads 16 are arranged in positions and postures to face the subject E so that the measurement optical axis L is positioned at the far-point position Pf (see FIG 16) of the bifocal lens Lb. In this posture, the ophthalmologic apparatus 10M performs the subjective examination by presenting the visual target at the far-point test distance with the visual target projection system 140 of the measurement optical system 20. This allows the ophthalmologic apparatus 10M to confirm the visibility in the direction of the far-point position Pf in the worn bifocal lens Lb.

Next, in the ophthalmologic apparatus 10M, both measurement heads 16 are arranged in a position and a posture to face the subject S wearing the bifocal lens Lb so that the measurement optical axis L is positioned at the near-point position Pn of the bifocal lens Lb (see FIG. 16) as illustrated in the lower side of FIG. 15. In this posture, the ophthalmologic apparatus 10M performs a subjective examination by presenting the visual target at the near-point test distance by the visual target projection system 140 of the measurement optical system 20. The ophthalmologic apparatus 10M thereby confirms the visibility in the direction of the near-point position Pn in the worn bifocal lens Lb.

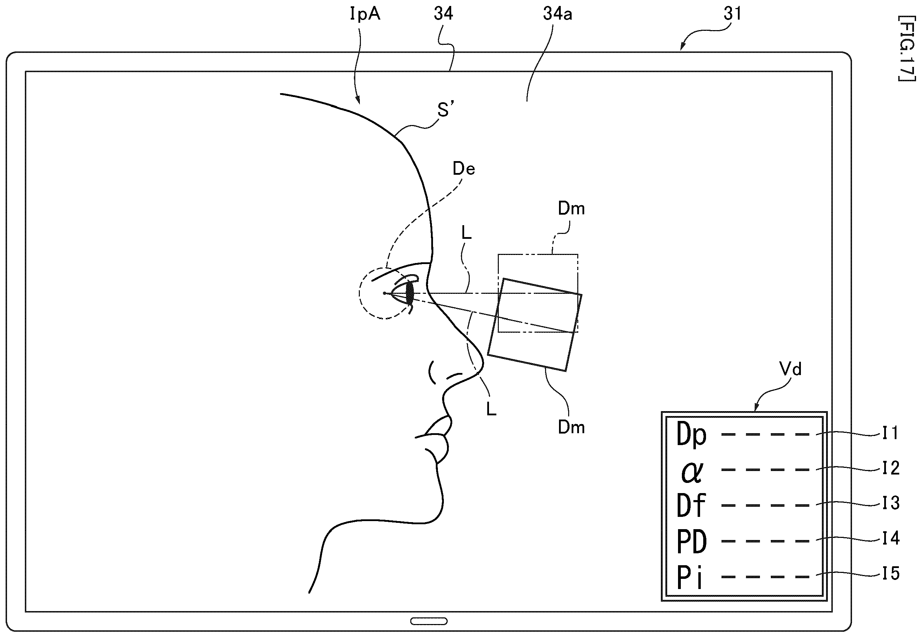

In the ophthalmologic apparatus 10M, the controller 27 creates the positional relationship image IpM (see FIG. 17) and displays it on the display 34 of the examiner's controller 31. The positional relationship image IpM illustrated in FIG. 17 is illustrated as seen from the left and right direction to easily understand the measurement optical system 20 (measurement head 16) is displaced in the up and down direction. In this positional relationship image IpM illustrated in FIG. 17, the solid line shows the measurement optical system 20 is arranged as illustrated in the lower side of FIG. 15 and the two-dot chain line shows the measurement optical system 20 is arranged as illustrated in the upper side of FIG. 15. The positional relationship image Ip in this example includes a pair of measurement optical system patterns Dm that imitates a pair of measurement optical systems 20, and a pair of subject eye patterns De that imitates a pair of subject eyes E. The controller 27 creates the positional relationship images IpM based on the optical posture information from the measurement head driver 40 as the optical system change mechanism and the subject eye positional information from each measurement optical system 20 as the acquisition optical system. In other words, the controller 27 finds the position and the orientation of each measurement optical system 20 with respect to each subject eye E based on the optical posture information and the subject eye position information. The controller 27 sets the position of a pair of subject eye patterns De based on the position and the orientation of each measurement optical system 20 and the position and the orientation of a pair of measurement optical system patterns Dm with respect to the position and the orientation of each measurement optical system 20 to create the positional relationship image IpM. Each measurement optical system patterns Dm displays the measurement optical axis L, which makes it easy to understand the orientation of each pattern. The positional relationship image IpA is provided with the various value display area Vd similar to the positional relationship image Ip illustrated in FIG. 12, for example. The controller 27 then displays the created positional relationship image IpM on the display 34 of the examiner's controller 31.

The positional relationship image IpM displays a pair of measurement head patterns Dh that imitates a pair of measurement heads 16 and a pair of deflection member patterns Dd that imitates a pair of deflection members 26 similar to the positional relationship image Ip illustrated in FIG. 12. In this case, the measurement head pattern Dh overlaps with the subject eye pattern De, which allows the display with a watermark or the subject eye pattern De to be visually recognized with a time difference. Therefore, the ophthalmologic apparatus 10M allows the examiner to easily understand the positional relationship of the measurement optical system 20 (measurement head 16) with respect to the subject eye E with the positional relationship image IpM.

The ophthalmologic apparatus 10M of the modified example shows the positional relationship image IpM in terms of the positional relationship of the subject eye E and the acquisition optical system (measurement optical system 20) as seen from the side, which allows the examiner to understand how the acquisition optical system is displaced to the subject eye E. Therefore, the ophthalmologic apparatus 10M allows the examiner to easily understand how the acquisition optical system (measurement optical system 20) is displaced in the vertical direction, and allows the examiner to objectively understand the positional relationship between the subject eye E and the acquisition optical system.

[Embodiment 2]

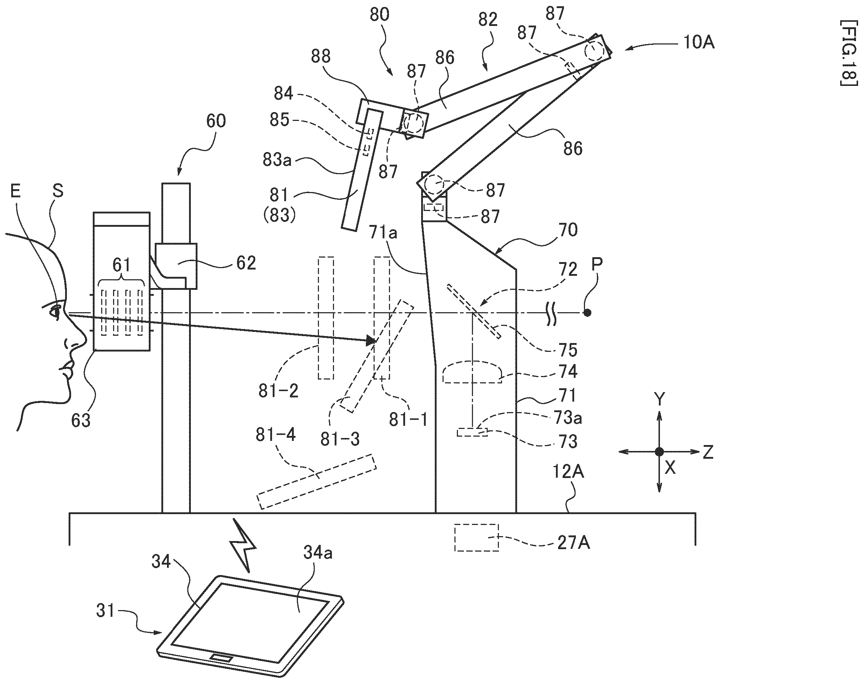

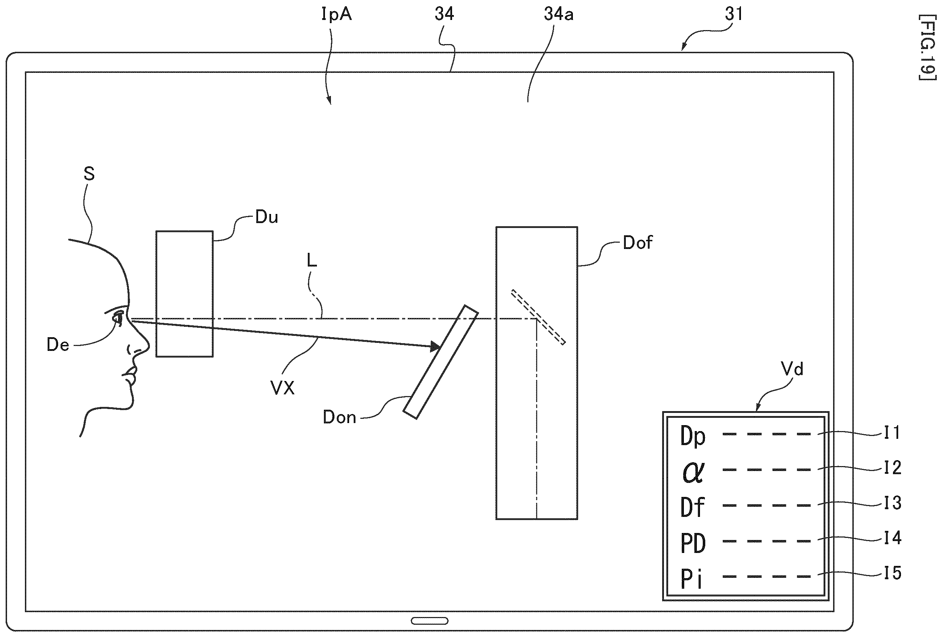

Next, an ophthalmologic apparatus 10A of Embodiment 2 of an embodiment of the present disclosure is described with reference to FIGS. 18, 19. As illustrated in FIG. 18, the ophthalmologic apparatus 10A includes an optometry table 12A, a controller 27A, an examiner's controller 31, a refractor head 60 that is an optometry optical system, a far-point visual target presentation device 70 for examining the visual function when the subject S visually observes a distant object (far-point test), and a near-point visual target presentation device 80 for examining the visual function when the subject S visually observes a near object (near-point test). The controller 27 totally controls the operation of the ophthalmologic apparatus 10A similar to the controller 27 of Embodiment 1. This controller 27A is provided on the optometry table 12A in the example illustrated in FIG. 18, but may be provided at any other location and is not limited to the configuration of Embodiment 2. The examiner's controller 31 can perform various operations of the refractor head 60, the far-point visual target presentation device 70 and the near-point visual target presentation device 80 under the control of the controller 27A similar to the ophthalmologic apparatus 10A of Embodiment 1.

The optometry table 12A is a desk on which the refractor head 60, the far-point visual target presentation device 70, the near-point visual target presentation device 80, and other devices are installed. The optometry table 12A is also used for the subject S to place his/her elbow or arm in order to maintain an appropriate posture during the examination, and is movable in the up and down direction. Therefore, the height of the refractor head 60, the far-point visual target presentation device 70, and the near-point visual target presentation device 80 can be adjusted in the ophthalmologic apparatus 10A according to the height of the subject eye E from a floor.

The refractor head 60 is a device that selects a lens that fits the subject eye E. The refractor head 60 includes a plurality of test lenses 61. The test lens 61 selected therefrom is placed in front of the subject eye E. The refractor head 60 thereby enables a refraction test and any other test to be performed while appropriately changing the test lens 61. Therefore, the refractor head 60 operates as a head portion with an acquisition optical system. The refractor head 60 includes a support mechanism 62 provided on the optometry table 12A and a pair of left and right optometry units 63 fixed at the end of the support mechanism.

Each of a pair of optometry units 63 includes a left eye optometry optical system and a right eye optometry optical system for the left and right subject eyes E of the subject S. A pair of optometry units 63 is provided to be individually slidable in the left and right direction (X direction) by a known sliding mechanism, and can relatively approach and separate from each other. The refractor head 60 can position a pair of optometry units 63 in front of or away from the front of the subject E by the rotation of the support mechanism 62 in the circumferential direction.

A front portion and a back portion of each optometry unit 63 are provided with optometry windows, respectively. Each optometry unit 63 includes inside thereof optical members such as a plurality of optical components such as a plurality of test lenses 61, polarizing filters, and shielding plates for the left and right subject eye E. These optical members can be selectively positioned by a known driving mechanism (not shown) at a position facing each optometry window. The test lens 61 is used to correct the visual function of the subject eye E. The test lens 61 includes a spherical lens, a cylindrical lens, and a prism. The test lens 61 can also be selected from a lens without a correction function, a glass plate, or a plastic plate in order to also allow an test with a naked eye.

The far-point visual target presentation device 70 presents a visual target for a far point (hereinafter also referred to as far-point visual target) through the test lens 61 of the refractor head 60 at the far-point test distance in front of the subject eye E. The far-point visual target presentation device 70 includes a housing 71 and a far-point visual target presentation optical system 72 housed therein. The front surface (subject S side) of the housing 71 is provided with a window 71a for the subject S to view the far-point visual target.

The far-point visual target presentation optical system 72 includes a display 73, a lens 74, and a reflecting mirror 75. The display 73 includes an electric display device such as an LCD or an organic EL. The display 73 displays a far-point visual target on its display surface 73a based on an instruction signal from the examiner's controller 31. The far-point visual target presentation optical system 72 refracts the light beam from the far-point visual target displayed on the display 73 by the lens 74 to image a visual target image (virtual image) P at the far-point test distance from the subject eye E. The light beam passed through the lens 74 is reflected by the reflecting mirror 75, and the visual target image (virtual image) P is presented at the far-point test distance in front of the subject eye E. The inclination angle of the reflecting mirror 75 can be changed, which allows the far-point visual target chart to present toward the subject eye E having variations in height depending on a height, for example. The far-point visual target presentation optical system 72 may include a magnifying lens or any other optical component as an test distance change mechanism, and can change the far-point test distance at which the visual target image (virtual image) P is presented by the test distance change mechanism according to an test purpose and an test content.

The near-point visual target presentation device 80 is used to examine the visual function of the subject S when the subject S views a near object (near-point test), and to finally confirm whether or not the prescription data obtained in the far-point test and the near-point test is appropriate and to slightly adjust the prescription data. The near-point visual target presentation device 80 includes a visual target display 81 and a visual target drive mechanism 82. The visual target display 81 is a tablet terminal integrally including (built-in) a visual target display portion 83, various sensors 84, and a visual target controller 85. The visual target display 81 may be a portable information device other than a tablet terminal such as a smart phone, a relatively small electric display device such as an LCD or an organic EL, or a visual target plate with a printed visual target, for example, as long as the visual target display 81 can present at least the visual target to the subject eye E and to move freely with the visual target drive mechanism 82. The visual target controller 85 controls the operation of the visual target display 81 and is capable of exchanging information with the controller 27A to control the display content of the visual target display 83 according to the instruction signal from the controller 27A. Furthermore, the visual target controller 85 outputs the detection data acquired by the various sensors 84 to the controller 27A in response to the instruction signal from the controller 27A.

The visual target display 83 has a display surface 83a and displays the visual target on the display surface 83a under the control of the visual target controller 85. The visual target display 83 may also display the results of the eye characteristics acquired by the ophthalmologic apparatus 10. The visual target display 83 includes an electric display device such as an LCD or an organic EL. The various sensors 84 include an acceleration sensor, a gyro sensor, a magnetic sensor, a GPS, and a distance measurement sensor.

The visual target drive mechanism 82 is provided on the top surface of the housing 71 of the far-point visual target presentation device 70. The visual target drive mechanism 82 three dimensionally moves the visual target display 81 in the XYZ directions to position the visual target display 81 (visual target displayed on display surface 83a of visual target display 83) in a desired position (e.g., test distance, height, left/right position) and a posture (inclination) with respect to the subject eye E. The visual target drive mechanism 82 has two arms 86, six rotation drivers 87, and a support 88. One arm 86 is attached to the top portion of the far-point visual target presentation device 70 via two rotation drivers 87. The visual target drive mechanism 82 may be mounted on the optometry table 12A or in another position, and is not limited to the configuration of Embodiment 2. A pair of rotation drivers 87 is provided at both ends of each arm 86 and is rotatable to about two orthogonal axes. Each rotation driver 87 includes a mechanism that holds the shaft body by a bearing, for example, and a motor that generates the driving force for each. The support 88 is an end effector (robot hand) and supports the visual target display 81.

The controller 27A obtains the current position information and the current posture information of the visual target display 81 (e.g., display surface 83a of visual target of visual target display unit 83) based on the information obtained by the various sensors 84 via the visual target controller 85 such as an acceleration, an angular velocity, an azimuth, position coordinates, a distance from a subject S, and an angle of a subject S's face to control the driving of the visual target drive mechanism 82 based on the position information and the posture information. The controller 27A uses the position coordinates at the initial position of a predetermined reference point (e.g., center position of display surface 83a) as an origin and controls the visual target drive mechanism 82 based on the test distance and the viewing object specified by the controller 31. Thus, the controller 27A can change the position and the posture of the visual target displayed on the display surface 83a of the visual target display 81 in relation to the subject eye E as desired, and can position the visual target at a predetermined position in the XYZ directions (left and right, up and down, and front and back) where its viewing object is located in a daily life.

Thereby, the visual target drive mechanism 82 appropriately positions the visual target indicator 81 in a desired position and a desired posture with respect to the subject eye E as illustrated by the dashed line in FIG. 18 under the control of the controller 27A. When the visual target drive mechanism 82 does not use the visual target display 81, the visual target drive mechanism 82 places the visual target display 81 in a predetermined evacuated position that does not face the subject eye E as illustrated by the solid line in FIG. 18 under the control of the controller 27A.

In Embodiment 2, the visual target drive mechanism 82 includes a six-axis robot arm, but it may also include a five-axis or seven-axis or more robot arm. In addition, although the visual target drive mechanism 82 includes a multi-joint type (vertically articulated) robot arm, which is one of serial link types, it is not limited to this configuration and may also include a horizontally articulated robot arm, a coordinate axis type robot arm (e.g., right angle coordinate, cylindrical coordinate, and curved coordinates),and a parallel link type robot. Furthermore, the visual target drive mechanism 82 may be manually moved as long as it can three dimensionally move the visual target indicator 81 to position in a desired position and a desired posture. When the visual target drive mechanism 82 moves manually, the controller 27A can acquires its three-dimensional position with a sensor that detects the three-dimensional position of the visual target display unit 81 moved by the visual target drive mechanism 82. The sensor may be the above-described various sensors 84.

The ophthalmologic apparatus 10A in Embodiment 2 can perform the far-point test and the near-point test of the subject eye E. First, the subject S (subject eye E) faces a pair of visual target units 63 located in front of the far-point visual target presentation device 70. When the refractor head 60 receives the instruction signal from the examiner's controller 31, the examination lens 61 is replaced and the power of the lens or the power of the prism placed in the optometry window is changed. Then, when the ophthalmologic apparatus 10A receives the instruction signal from the examiner's controller 31, the ophthalmologic apparatus 10A controls the far-point visual target presentation device 70 under the control of the controller 27A to present the visual target in a predetermined display manner.

For starting the far-point test, the examiner's controller 31 is operated to select the far-point test. With this operation, the far-point visual target presentation device 70 displays the far-point visual target on the display surface 73a of the display 73. The subject S can perform the subjective far-point test by viewing the far-point visual target presented to the subject eye E by the far-point visual target presentation optical system 72 through the optometry unit 63. In the ophthalmologic apparatus 10A, the examiner's controller 31 is operated according to the vision of the subject S to appropriately select the test lens 61 to be placed in the visual target window (in front of subject eye E) so that the subject S repeats the far-point test. As a result, the examiner can correct the adjustment state, the heterophoria, etc., of the subject eye E when the subject S views the far-point object, and acquires the prescription for the far-point correction.

Next, for the near-point test, the examiner operates the examiner's controller 31 to drive the visual target drive mechanism 82 to position the visual target display 81 at a position corresponding to the height of the subject eye E (e.g., position illustrated by reference number 81-1 in FIG. 18), and the visual target display 83 displays the near-point visual target on the display 83a. The subject S views the near-point visual target presented to the subject eye E through the optometry unit 63 so that the subject S can perform the subjective examination. At this time, the examiner operates the examiner's controller 31 to drive the visual target drive mechanism 82 to change the position of the visual target display 81 in the Z direction and change the near-point test distance as desired (e.g., position indicated by reference number 81-2 in FIG. 18).

Furthermore, in the ophthalmologic apparatus 10A, the examiner operates the examiner's controller 31 according to the vison of the subject S and other factors to appropriately select the test lens 61 to be placed in the ophthalmologic window (in front of subject eye E) so that the subject S repeats the near-point test. As a result, the examiner can correct the adjustment state, the heterophoria, etc., of the subject eye E when viewing the near-point object, and acquire the prescription for the near-point correction.

Once the far-point prescription and the near-point prescription have been determined, the examiner's controller 31 is operated to designate the test distance or the viewing object using icons, for example, in order to finally confirm the vision. In response to this designation, the visual target drive mechanism 82 positions the visual target indicator 81 in a position and a posture corresponding to the test distance or a position and a posture of the viewing object (e.g., position indicated by reference number 81-3 and 81-4 in FIG. 18). The subject S looks at the near-point visual target through the optometry unit 63 while moving the visual line up and down and left and right in response to the direction of the near-point visual target presented by the visual target display 81. This allows the subject S to finally confirm the visual performance in a situation closer to a real life. During the final confirmation, the subject S may wear a trial frame with inspection lenses attached, finished spectacles or contact lenses instead of the refractor head 60.

Therefore, in the ophthalmologic apparatus 10A, the refractor head 60, the far-point visual target presentation device 70, and the near-point visual target presentation device 80 function as the acquisition optical system for acquiring the eye information of both subject eyes E. The refractor head 60, the far-point visual target presentation device 70, and the visual target drive mechanism 82 function as the optical system change mechanism for changing at least one of the positions of the refractor head 60 as the acquisition optical system and the position or the orientation of the near-point visual target presentation device 80. The interval between a pair of optometry units 63 and the position and the orientation of the near-point visual target presentation device 80 are used as the optical posture information indicating the position and the orientation of the acquisition optical system. In addition, the information of the interval of a pair of optometry units 63 and the position and the orientation of the near-point visual target presentation device 80 with respect to the subject eye E are used as the subject eye position information indicating the orientation of the subject eye E.

In the ophthalmologic apparatus 10A of Embodiment 2, the controller 27A creates the positional relationship image IpA (see FIG. 19) and displays it on the display 34 of the examiner's controller 31. The positional relationship image IpA illustrated in FIG. 19 shows the visual performance is finally confirmed as described above with respect to both subject eyes E and the visual target indicator 81 is positioned as illustrated by the reference number 81-3 in FIG. 18. This positional relationship image Ip is illustrated as viewed from the left and right direction, which makes it easy to understand how the visual target display 81 is displaced in the vertical direction. The positional relationship image IpA in FIG. 19 includes a subject eye pattern De that imitates the subject eye E, an optometry unit pattern Du that imitates the optometry unit 63, a far-point visual target presentation pattern Dof that imitates the far-point visual target presentation device 70, and a near-point visual target presentation pattern Don that imitates the visual target display 81. The controller 27 sets the position of the subject eye pattern De and the position and the orientation of the optometry unit pattern Du, the far-point visual target presentation pattern Dof and the near-point visual target presentation pattern Don based on the optical posture information and the eye position information described above to create the positional relationship image IpA. The positional relationship image IpA includes the various value display area Vd similar to the positional relationship image Ip of Embodiment 1. Therefore, in the ophthalmologic apparatus 10A, the positional relationship of the optometry unit 63, the far-point visual target presentation device 70, and the visual target display 81 to the subject eye E can be easily understood by the positional relationship image IpA.

The ophthalmologic apparatus 10A of Embodiment 2 of the ophthalmologic apparatus according to the present disclosure can acquire the following effects. The ophthalmologic apparatus 10A includes basically the same configuration as the ophthalmologic apparatus 10 of Embodiment 1, and thus, the ophthalmologic apparatus 10A can acquire the same effects as in Embodiment 1.

In addition to that, the ophthalmologic apparatus 10A shows the positional relationship image IpA with the positional relationship as viewed the subject eye E and the acquisition optical system (refractor head 60, far-point visual target presentation device 70, and near-point visual target presentation device 80) from the side, which allows the examiner to understand how the acquisition optical system is displaced in the vertical direction relative to the subject eye E. Therefore, the ophthalmologic apparatus 10A makes it easy to understand how the acquisition optical system (near-point visual target presentation device 80) is displaced in the up and down direction and allows the examiner to objectively understood the positional relationship between the subject eye E and the acquisition optical system.

Accordingly, in the ophthalmologic apparatus 10A of Embodiment 2 of the ophthalmologic apparatus according to the present disclosure even when the subject places his/her face against the refractor head 60 as the head part, the positional relationship between the subject eye E and the refractor head 60 as the acquisition optical system, the far-point visual target presentation device 70, and the near-point visual target presentation device 80 can be objectively understood.

Although the ophthalmologic apparatus of the present disclosure is described based on each embodiment, the specific configuration is not limited to each embodiment, and design changes, additions, etc., are permitted as long as they do not depart from the gist of the invention claimed in each claim.

For example, each example (including modified example) shows the example of the positional relationship image Ip of Embodiment 1 illustrated in FIGS. 12, 13 of Embodiment 1, the example of the positional relationship image IpM of the modified example illustrated in FIG. 17, and the example of the positional relationship image IpA of Embodiment 2 illustrated in FIG. 19, as the positional relationship image, but the examples are not limited to each example as long as they show the positional relationship of a pair of acquisition optical systems (measurement optical system 20) to the subject eye E. For example, the positional relationship image of Embodiment 1 may be a simplified image showing the subject eye and the optical system as illustrated in FIG 3 or may be an image schematically showing the position displayed by the acquisition optical system with respect to the subject eye as shown on the left side of FIG. 8 or FIG. 9. For example, the positional relationship image in FIG. 17 of the modified example may also be an image showing the measurement head driver supports the measurement head as shown in FIG. 15, may be an image showing the subject eye and optical system in more detail, or may be an image showing the subject eye and optical system in a simplified manner. Furthermore, for example, the positional relationship image of Embodiment 2 may be an image showing the subject eye and optical system in more detail as shown in FIG. 18, may be an image show the subject eye and optical system in a simplified manner, or may be an image schematically showing the position of the acquisition optical system with respect to the subject eye.

Each example (including modified example) shows the example of the positional relationship image Ip of Embodiment 1 illustrated in FIGS. 12, 13, the example of the positional relationship image IpM of the modified example illustrated in FIG. 17, and the example of the positional relationship image IpA of Embodiment 2 illustrated in FIG. 19 as the positional relationship images. The positional relationship image Ip is shown in the positional relationship looking down from above, while the positional relationship image IpM and the positional relationship image IpA are shown in the positional relationship looking from the side. However, the positional relationship image Ip is not limited to the configuration of Embodiment 1 as long as it enables at least the understanding of the horizontal displacement of a pair of acquisition optical systems with respect to the subject eye E. In addition, the positional relationship image IpM and the positional relationship image IpA are not limited to the configurations of the modified example and Embodiment 2 as long as they enable at least understanding of the vertical displacement of a pair of acquisition optical systems with respect to the subject eye E. This positional relationship image may include a three-dimensional view showing the positional relationship of a pair of acquisition optical systems with respect to the subject eye E. This three-dimensional view may be shown, for example, with the third angle method, the first angle method, the isometric projection method, the oblique projection method, or the perspective projection method. When the positional relationship image is made into the three-dimensional view in this way, both the horizontal displacement of a pair of acquisition optical systems with respect to the subject eye E and the vertical displacement of a pair of acquisition optical systems with respect to the subject eye E can be understood.

Furthermore, each example (including modified example), in the ophthalmologic apparatuses 10, 10A, the subject S operates the examiner's controller 31. However, an external examiner in a room different from the room in which the ophthalmologic apparatuses 10, 10A are installed may operate the external controller, and the configuration is not limited to the configuration of each example. In this case, both the examiner's controller 31 and the external controller may be available.

Each example (including modified example) shows as Embodiment 1 each measurement optical system 20 as the acquisition optical system and as Embodiment 2 the refractor head 60, the far-point visual target presentation device 70, and the near-point visual target presentation device 80. However, the acquisition optical system is not limited to the configuration in each example as long as is provided in pairs for acquiring the eye information of both subject eyes E. The optical system change mechanism is not limited to the configuration of each example as long as it changes at least appropriately set one of the position or the orientation of a pair of acquisition optical systems.

CROSS-REFERENCE TO RELATED APPLICATIONS

This application claims priority based on Japanese Patent Application No. 2022-155540 filed with the Japan Patent Office on September 28, 2022, the entire disclosure of which is entirely incorporated herein by reference.

**Claims**

1. An ophthalmologic apparatus,

   a pair of acquisition optical systems that are configured to acquire eye information of both subject eyes;

an optical system change mechanism that is configured to change at least one of a position or an orientation of the pair of acquisition optical systems; and
a controller that is configured to create a positional relationship image showing a positional relationship of the pair of acquisition optical systems with respect to a pair of subject eyes based on optical posture information indicating the position and the orientation of the pair of acquisition optical systems set by the optical system change mechanism and subject eye position information indicating a position of the subject eye, and displays the positional relationship image on a display.

2. The ophthalmologic apparatus according to claim 1, wherein the positional relationship image includes information of a measurement optical axis in the pair of acquisition optical systems.

3. The ophthalmologic apparatus according to claim 1 or claim 2, wherein the subject eye position information is alignment information in the acquisition optical system.

4. The ophthalmologic apparatus according to any one of claims 1 to 3, wherein the positional relationship image includes proximity degree information indicating a proximity degree from the pair of acquisition optical systems to a corresponding subject eye.

5. The ophthalmologic apparatus according to any one of claims 1 to 4, wherein the positional relationship image enables understanding of horizontal displacement of the pair of acquisition optical systems with respect to the pair of subject eyes.

6. The ophthalmologic apparatus according to any one of claims 1 to 5, wherein the positional relationship image enables understanding of vertical displacement of the pair of acquisition optical systems with respect to the pair of subject eyes.

7. The ophthalmologic apparatus according to any one of claims 1 to 6, wherein the positional relationship image includes at least one of interval information indicating an interval between the pair of subject eyes, focusing distance information indicating a focusing distance of a visual target image presented by the pair of acquisition optical systems, and rotation angle information indicating a rotation angle of the pair of acquisition optical systems.

【FIG.1】

[FIG.2]

[FIG.3]

[FIG.4]

EP 4 595 870 A1

[FIG.5]

[FIG.6]

163

163b

163a

165

[FIG.7]

165

164

[FIG.8]

[FIG.9]

[FIG.10]

[FIG.11]

[FIG.12]

EP 4 595 870 A1

EP 4 595 870 A1

[FIG.13]

[FIG.14]

[FIG.15]

[FIG.16]

[FIG.17]

[FIG.18]

[FIG.19]

## INTERNATIONAL SEARCH REPORT

| International application No. |
| --- |
| **PCT/JP2023/034003** |

| A. | CLASSIFICATION OF SUBJECT MATTER |
| --- | --- |

*A61B 3/08*(2006.01)i
FI:  A61B3/08

According to International Patent Classification (IPC) or to both national classification and IPC

| B. | FIELDS SEARCHED |
| --- | --- |

Minimum documentation searched (classification system followed by classification symbols)

A61B3/00-3/18

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2020-54784 A (TOPCON CORP) 09 April 2020 (2020-04-09)<br>      paragraphs [0015], [0027], [0077]-[0078], [0086], [0088], [0090], fig. 15 | 1-7 |
| Y | WO 2022/091209 A1 (RIVERFIELD INC) 05 May 2022 (2022-05-05)<br>      paragraphs [0020]-[0026], [0048]-[0056], [0073] | 1-7 |
| Y | JP 2018-38518 A (TOPCON CORP) 15 March 2018 (2018-03-15)<br>      paragraphs [0011], [0053], fig. 4 | 1-7 |
| A | JP 2020-127588 A (TOPCON CORP) 27 August 2020 (2020-08-27)<br>      paragraph [0064] | 1-7 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 October 2023** | **12 December 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/034003**

| Patent document cited in search report | | | Publication date (day/month/year) | Patent family member(s) | | | Publication date (day/month/year) |
|---|---|---|---|---|---|---|---|
| JP | 2020-54784 | A | 09 April 2020 | JP | 2023-24761 | A | |
| WO | 2022/091209 | A1 | 05 May 2022 | CN | 116133571 | A | |
| JP | 2018-38518 | A | 15 March 2018 | JP | 2021-164840 | A | |
| JP | 2020-127588 | A | 27 August 2020 | US | 2020/0253472 | A1 | |
| | | | | paragraph [0072] | | | |
| | | | | EP | 3692891 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003041571 A
- JP 2022155540 A